# EUROPÄISCHE PATENTSCHRIFT

(11) **EP 1 119 557 B1**
(45) Veröffentlichungstag und Bekanntmachung des Hinweises auf die Patenterteilung: **03.12.2003**
(21) Anmeldenummer: 99969717.0
(22) Anmeldetag: 16.09.1999
(51) Int. Cl.: C07D 277/60, C07D 417/04, C07D 417/12, C07D 417/10, C07D 417/06, C07D 417/14, A61P 3/04, A61K 31/425, A61K 31/44

(54) **INDENO-, NAPHTHO- UND BENZOCYCLOHEPTA-DIHYDROTHIAZOL DERIVATE, DEREN HERSTELLUNG UND DEREN VERWENDUNG ALS ANOREKTISCHE ARZNEIMITTEL**
INDENO-, NAPHTO- AND BENZOCYCLOHEPTA DIHYDROTHIAZOLE DERIVATIVES, THE PRODUCTION THEREOF AND THEIR USE AS ANORECTIC MEDICAMENTS
DERIVES INDENO-, NAPHTO- ET BENZOCYCLOHEPTA-DIHYDROTHIAZOL, LEUR FABRICATION ET LEUR UTILISATION COMME MEDICAMENTS ANOREXIGENES

(30) Priorität: 29.09.1998 DE 19844547
(43) Veröffentlichungstag der Anmeldung: 01.08.2001
(73) Patentinhaber: Aventis Pharma Deutschland GmbH, 65929 Frankfurt am Main (DE)
(72) Erfinder: JÄHNE, Gerhard, D-65929 Frankfurt am Main (DE); GLOMBIK, Heiner, D-65719 Hofheim (DE); GEISEN, Karl, D-60318 Frankfurt am Main (DE); BICKEL, Martin, D-61348 Bad Homburg (DE)
(86) Internationale Anmeldenummer: EP9906860
(87) Internationale Veröffentlichungsnummer: WO00018749

(56) Entgegenhaltungen:
- EP-A- 0 749 966
- WO-A-97/08159
- WO-A-98/13356
- DE-A- 2 640 358
- US-A- 2 942 003
- CHEMICAL ABSTRACTS, vol. 73, no. 9, 31. August 1970 (1970-08-31) Columbus, Ohio, US; abstract no. 45502p, YAMANE K.: "Benzocycloheptathiazoles" Seite 325; Spalte 1; XP002018029 & JP 45 014068 A (JAPANESE RESEARCH INSTITUTE FOR PHOTOSENSITIZING DYES CO., LTD.) 20. Mai 1970 (1970-05-20)
- HASHEM M.M. ET AL.: "Novel pyrazolo, isoxazolo, and thiazolo steroidal systems and model analogs containing dimethoxylaryl (or dihydroxylaryl) groups and derivatives. Synthesis spectral properties, and biological activity" JOURNAL OF MEDICINAL CHEMISTRY, Bd. 19, Nr. 2, Februar 1976 (1976-02), Seiten 229-239, XP000609112
- PERRONE R. ET AL.: "Conformationally restricted thiazole derivatives as novel class of 5-HT3 receptor ligands" IL FARMACO, Bd. 50, Nr. 2, Februar 1995 (1995-02), Seiten 77-82, XP000605205 ISSN: 0014-827X
- CHEMICAL ABSTRACTS, vol. 120, no. 2, 10. Januar 1994 (1994-01-10) Columbus, Ohio, US; abstract no. 14653y, SAIDA M. ET AL.: "Preparation of thiazoles as tyrosinase inhibitors and skin-lightening preparations containing them" Seite 421; Spalte 1; XP002123115 & JP 05 222015 A (HISAMITSU PHARMACEUTICAL CO.) 31. August 1993 (1993-08-31)

## Beschreibung

Die Erfindung betrifft polycyclische Dihydrothiazole sowie deren physiologisch verträgliche Salze.

Es sind bereits Thiazolidinderivate mit anorektischer Wirkung im Stand der Technik beschrieben (Österreichisches Patent Nr. 365181).

Der Erfindung lag die Aufgabe zugrunde, weitere Verbindungen zur Verfügung zu stellen, die eine therapeutisch verwertbare anorektische Wirkung entfalten.

Die Erfindung betrifft daher Verbindungen der Formel I, 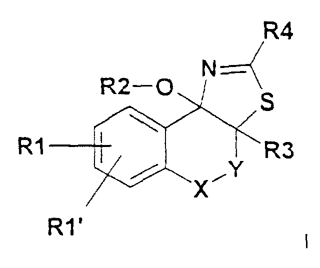 worin bedeuten
- Y: eine direkte Bindung, -CH₂-, -CH₂-CH₂-;
- X: CH₂, CH(CH₃), CH(C₂H₅), CH(C₃H₇), CH(C₆H₅);
- R1, R1': unabhängig voneinander H, F, Cl, Br, J, CF₃, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können, oder ein Wasserstoff durch OH,
OC(O)CH₃, OC(O)H, OCH₂Ph, NH₂,NH-CO-CH₃ oder
N(COOCH₂Ph)₂ ersetzt sein kann; SO₂-NH₂, SO₂NH(C₁-C₆)-Alkyl, SO₂N[(C₁-C₆)-Alkyl]₂, S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; NH₂, NH-(C₁-C₆)-Alkyl, N((C₁-C₆)-Alkyl)₂, NH(C₁-C₇)-Acyl, Phenyl, Biphenyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenyl-, Naphthyl-, Pyridyl-, Furanyl- oder Thienylringe jeweils ein bis 3-fach substituiert sein können mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
- R2: H, (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, C(O)-(C₁-C₆)-Alkyl, C(O)-(C₃-C₆)-Cycloalkyl, C(O)-(CH₂)ₙ-Phenyl, C(O)-(CH₂)ₙ-Thienyl, C(O)-(CH₂)ₙ-Pyridyl, C(O)-(CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann;
- R3: H, (C₁-C₆)-Alkyl, F, CN, N₃, O-(C₁-C₆)-Alkyl, (CH₂)ₙ-Phenyl, (CH₂)ₙ-Thienyl, (CH₂)ₙ-Pyridyl, (CH₂)ₙ-Furyl, wobei n = 0 - 5 sein kann; und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann; (C₂-C₆)-Alkinyl, (C₂-C₆)-Alkenyl, C(O)OCH₃, C(O)OCH₂CH₃, C(O)OH, C(O)NH₂, C(O)NHCH₃, C(O)N(CH₃)₂, OC(O)CH₃;
- R4: (C₁-C₈)-Alkyl, (C₃-C₇)-Cycloalkyl, (C₂-C₆)-Alkenyl, (C₂-C₆)-Alkinyl, (C₄-C₇)-Cycloalkenyl, wobei in den Alkylresten ein, mehrere, oder alle Wasserstoff(e) durch Fluor ersetzt sein können oder ein Wasserstoff durch OH, OC(O)CH₃, OC(O)H,O-CH₂-Ph oder O-(C₁-C₄)-Alkyl ersetzt sein kann;
(CH₂)ₙ-NR6R7, wobei n = 1-6 sein kann und R6 und R7 unabhängig voneinander H, (C₁-C₆)-Alkyl, (C₃-C₆)-Cycloalkyl, CO-(C₁-C₆)-Alkyl, CHO oder CO-Phenyl sein können, oder -NR6R7 stellt einen Ring wie Pyrrolidin, Piperidin, Morpholin. Piperazin, N-4-Methyl-piperazin-1-yl, N-4-Benzyl-piperazin-1-yl, Phthalimidoyl dar;
(CH₂)n-Aryl, wobei n = 0 - 6 sein kann und Aryl Phenyl, Biphenyl, 1- oder 2-Naphthyl, 2-, 3- oder 4-Pyridyl, 2- oder 3-Thienyl, 2- oder 3-Furyl, 2-, 4- oder 5-Thiazolyl, 2-, 4- oder 5-Oxazolyl, 1-Pyrazolyl, 3- oder 5-Isoxazolyl, 2- oder 3-Pyrrolyl, 2- oder 3-Pyridazinyl, 2-, 4- oder 5-Pyrimidinyl, 2-Pyrazinyl, 2-(1,3,5-Triazinyl), 2- oder 5-Benzimidazolyl, 2-Benzothiazolyl, 1,2,4-Triazal-3-yl, 1,2,4-Triazol-5-yl, Tetrazol-5-yl, Indol-3-yl, Indol-5-yl oder N-Methyl-imidazol-2-, -4- oder -5-yl sein kann und der Arylrest oder Heteroarylrest bis zu zweifach mit F, Cl, Br, OH, CF₃, NO₂. CN, OCF₃, O-(C₁-C₆)-Alkyl, S-(C₁-C₆)-Alkyl, SO-(C₁-C₆)-Alkyl, SO₂-(C₁-C₆)-Alkyl, (C₁-C₆)-Alkyl, (C₃-C₆)-Cycloalkyl, COOH, COO(C₁-C₆)Alkyl, COO(C₃-C₆)Cycloalkyl, CONH₂, CONH(C₁-C₆)Alkyl, CON[(C₁-C₆)Alkyl]2, CONH(C₃-C₆)Cycloalkyl, NH₂, NH-CO-(C₁-C₆)-Alkyl, NH-CO-Phenyl, Pyrrolidin-1-yl, Morpholin-1-yl, Piperidin-1-yl, Piperazin-1-yl, 4-Methyl-piperazin-1-yl, (CH₂)ₙ-Phenyl, O-(CH₂)ₙ-Phenyl, S-(CH₂)ₙ-Phenyl, SO₂-(CH₂)ₙ-Phenyl, wobei n = 0-3, substituiert sein kann;
sowie deren physiologisch verträgliche Salze.

Bevorzugt sind Verbindungen der Formel I, in denen ein oder mehrere Rest(e), die folgende Bedeutung hat bzw. haben:
- Y: eine direkte Bindung;
- X: CH₂;
- R1, R1': unabhängig voneinander H, F, Cl, Br, J, CF₃, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können, oder ein Wasserstoff durch OH, OC(O)CH₃, OC(O)H, O-CH₂-Ph, NH₂, NH-CO-CH₃ oder N(COOCH₂Ph)₂ ersetzt sein kann;
SO₂-NH₂, SO₂NH(C₁-C₆)-Alkyl, SO₂N[(C₁-C₆)-Alkyl]₂, S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; NH₂, NH-(C₁-C₆)-Alkyl, N((C₁-C₆)-Alkyl)₂, NH(C₁-C₇)-Acyl, Phenyl, Biphenyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenyl-, Naphthyl-, Pyridyl-, Furanyl- oder Thienylringe jeweils ein bis 3-fach substituiert sein können mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C1-C₆)-Alkyl, CONH₂;
1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
- R2: H, (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, C(O)-(C₁-C₆)-Alkyl, C(O)-(C₃-C₆)-Cycloalkyl, C(O)-(CH₂)ₙ-Phenyl, C(O)-(CH₂)ₙ-Thienyl, C(O)-(CH₂)ₙ-Pyridyl, C(O)-(CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann;
- R3: H, (C₁-C₆)-Alkyl, F, CN, N₃, O-(C₁-C₆)-Alkyl, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann, (C₂-C₆)-Alkinyl, (C₂-C₆)-Alkenyl, C(O)OCH₃, C(O)OCH₂CH₃, C(O)OH, C(O)NH₂, C(O)NHCH₃, C(O)N(CH₃)₂, OC(O)CH_{3;}
- R4: (C₁-C₈)-Alkyl, (C₃-C₇)-Cycloalkyl, (C₂-C₆)-Alkenyl, (C₂-C₆)-Alkinyl, (C₄-C₇)-Cycloalkenyl, wobei in den Alkylresten ein, mehrere, oder alle Wasserstoff(e) durch Fluor ersetzt sein können oder ein Wasserstoff durch OH, OC(O)CH₃, OC(O)H,O-CH₂-Ph oder O-(C₁-C₄)-Alkyl ersetzt sein kann;
(CH₂)n-NR6R7, wobei n = 1-6 sein kann und R6 und R7 unabhängig voneinander H, (C₁-C₆)-Alkyl, (C₃-C₆)-Cycloalkyl, CO-(C₁-C₆)-Alkyl, CHO oder CO-Phenyl sein können, oder -NR6R7 stellt einen Ring wie Pyrrolidin, Piperidin, Morpholin, Piperazin, N-4-Methyl-piperazin-1-yl, N-4-Benzyl-piperazin-1-yl, Phthalimidoyl dar;
(CH₂)ₙ-Aryl, wobei n = 0 - 6 sein kann und Aryl Phenyl, Biphenyl, 1- oder 2-Naphthyl, 2-, 3- oder 4-Pyridyl, 2- oder 3-Thienyl, 2- oder 3-Furyl, 2-, 4- oder 5-Thiazolyl, 2-, 4- oder 5-Oxazolyl, 1-Pyrazolyl, 3- oder 5-Isoxazolyl, 2- oder 3-Pyrrolyl, 2- oder 3-Pyridazinyl, 2-, 4- oder 5-Pyrimidinyl, 2-Pyrazinyl, 2-(1,3,5-Triazinyl), 2- oder 5-Benzimidazolyl, 2-Benzothiazolyl, 1,2,4-Triazol-3-yl, 1,2,4-Triazol-5-yl, Tetrazol-5-yl, Indol-3-yl, Indol-5-yl oder N-Methyl-imidazol-2-, 4- oder -5-yl sein kann und der Arylrest oder Heteroarylrest bis zu zweifach mit F, Cl, Br, OH, CF₃, NO₂, CN, OCF₃, O-(C₁-C₆)-Alkyl, S-(C₁-C₆)-Alkyl, SO-(C₁-C₆)-Alkyl, SO₂-(C₁-C₆)-Alkyl, (C₁-C₆)-Alkyl, (C₃-C₆)-Cycloalkyl, COOH, COO(C₁-C₆)Alkyl, COO(C₃-C₆)Cycloalkyl, CONH₂, CONH(C₁-C₆)Alkyl, CON[(C₁-C₆)Alkyl]2, CONH(C₃-C₆)Cycloalkyl, NH₂, NH-CO-(C₁-C₆)-Alkyl, NH-CO-Phenyl, Pyrrolidin-1-yl, Morpholin-1-yl, Piperidin-1-yl, Piperazin-1-yl, 4-Methyl-piperazin-1-yl, (CH₂)ₙ-Phenyl, O-(CH₂)ₙ-Phenyl, S-(CH₂)ₙ-Phenyl, SO₂-(CH₂)ₙ-Phenyl, wobei n = 0-3, substituiert sein kann;
sowie deren physiologisch verträgliche Salze.

Besonders bevorzugt sind Verbindungen der Formel I, in denen ein oder mehrere Rest(e), die folgende Bedeutung hat bzw. haben:
- Y: eine direkte Bindung;
- X: CH₂;
- R1, R1': unabhängig voneinander H, F, Cl, Br, J, CF3, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können, oder ein Wasserstoff durch OH, OC(O)CH₃, OC(O)H, O-CH₂-Ph, NH₂, NH-CO-CH₃ oder N(COOCH₂Ph)₂ ersetzt sein kann;
SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, Biphenyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenyl-, Naphthyl-, Pyridyl-, Furanyl- oder Thienylringe jeweils ein bis 3-fach substituiert sein können mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂,
1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
- R2: H, (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, C(O)-(C₁-C₆)-Alkyl, C(O)-(C₃-C₆)-Cycloalkyl, C(O)-(CH₂)ₙ-Phenyl, C(O)-(CH₂)ₙ-Thienyl, C(O)-(CH₂)ₙ-Pyridyl, C(O)-(CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann;
- R3: H, F;
- R4: (C₁-C₈)-Alkyl, (C₃-C₇)-Cycloalkyl, (C₂-C₆)-Alkenyl, (C₂-C₆)-Alkinyl, (C₄-C₇)-Cycloalkenyl, wobei in den Alkylresten ein, mehrere, oder alle Wasserstoff(e) durch Fluor ersetzt sein können oder ein Wasserstoff durch OH, OC(O)CH₃, OC(O)H,O-CH₂-Ph oder O-(C₁-C₄)-Alkyl ersetzt sein kann;
(CH₂)n-NR6R7, wobei n = 1-6 sein kann und R6 und R7 unabhängig voneinander H, (C₁-C₆)-Alkyl, (C₃-C₆)-Cycloalkyl, CO-(C₁-C₆)-Alkyl, CHO oder CO-Phenyl sein können, oder -NR6R7 stellt einen Ring wie Pyrrolidin, Piperidin, Morpholin, Piperazin, N4-Methyl-piperazin-1-yl, N4-Benzyl-piperazin-1-yl, Phthalimidoyl dar;
(CH₂)ₙ-Aryl, wobei n = 0 - 6 sein kann und Aryl Phenyl, Biphenyl, 1- oder 2-Naphthyl, 2-, 3- oder 4-Pyridyl, 2- oder 3-Thienyl, 2- oder 3-Furyl, 2-, 4- oder 5-Thiazolyl, 2-, 4- oder 5-Oxazolyl, 1-Pyrazolyl, 3- oder 5-Isoxazolyl, 2- oder 3-Pyrrolyl, 2- oder 3-Pyridazinyl, 2-, 4- oder 5-Pyrimidinyl, 2-Pyrazinyl, 2-(1,3,5-Triazinyl), 2- oder 5-Benzimidazolyl, 2-Benzothiazolyl, 1,2,4-Triazol-3-yl, 1,2,4-Triazol-5-yl, Tetrazol-5-yl, Indol-3-yl, Indol-5-yl oder N-Methyl-imidazol-2-, 4- oder -5-yl sein kann und der Arylrest oder Heteroarylrest bis zu zweifach mit F, Cl, Br, OH, CF₃, NO₂, CN, OCF₃, O-(C₁-C₆)-Alkyl, S-(C₁-C₆)-Alkyl, SO-(C₁-C₆)-Alkyl, SO₂-(C₁-C₆)-Alkyl, (C₁-C₆)-Alkyl, (C₃-C₆)-Cycloalkyl, COOH, COO(C₁-C₆)Alkyl, COO(C₃-C₆)Cycloalkyl, CONH₂, CONH(C₁-C₆)Alkyl, CON[(C₁-C₆)Alkyl]₂, CONH(C₃-C₆)Cycloalkyl, NH₂, NH-CO-(C₁-C₆)-Alkyl, NH-CO-Phenyl, Pyrrolidin-1-yl, Morpholin-1-yl, Piperidin-1-yl, Piperazin-1-yl, 4-Methyl-piperazin-1-yl, (CH2)ₙ-Phenyl, O-(CH₂)ₙ-Phenyl, S-(CH₂)ₙ-Phenyl, SO₂-(CH₂)ₙ-Phenyl, wobei n = 0-3, substituiert sein kann;
sowie deren physiologisch verträgliche Salze.

Ganz besonders bevorzugt sind Verbindungen der Formel I, in denen ein oder mehrere Rest(e), die folgende Bedeutung hat bzw. haben:
- Y: eine direkte Bindung;
- X: CH₂;
- R1, R1': unabhängig voneinander H, F, Cl, Br, NO₂, CN, COOH, (C₁-C₆)-Alkyl, (C₂-C₆)-Alkenyl, (C₂-C₆)-Alkinyl, O-(C₁-C₆)-Alkyl, OCF₃, OCH₂CF₃, Phenyl, O-(CH₂)ₙ-Phenyl, wobei n = 0 - 6 sein kann, 1- oder 2-Naphthyl, 2- oder 3-Thienyl, wobei die Phenyl-, Naphthyl- oder Thienylringe jeweils ein bis 3-fach substituiert sein können mit F, Cl, Br, J, OH, CF₃, NO₂, CN, OCF₃, O-(C₁-C₆)-Alkyl, (C₁-C₆)-Alkyl, NH₂;
- R2: H, (C₁-C₆)-Alkyl, (C₃-C₆)-Cycloalkyl, (CH₂)ₙ-Phenyl, wobei n = 0-5 sein kann;
- R3: H, F;
- R4: (C₁-C₈)-Alkyl, (C₃-C₇)-Cycloalkyl, (CH₂)ₙ-NR6R7, wobei n = 1-6 sein kann und R6 und R7 unabhängig voneinander H, (C₁-C₆)-Alkyl sein können, oder -NR6R7 stellt einen Ring wie Pyrrolidin, Piperidin, Morpholin, Piperazin, N4-Methyl-piperazin-1-yl, N4-Benzyl-piperazin-1-yl, Phthalimidoyl dar;
(CH₂)ₙ-Aryl, wobei n = 0 - 6 sein kann und Aryl Phenyl, Biphenyl, 1- oder 2-Naphthyl, 2-, 3- oder 4-Pyridyl, 2- oder 3-Thienyl, 2- oder 3-Furyl, 2-, 4- oder 5-Thiazolyl, 2-, 4- oder 5-Oxazolyl, 1-Pyrazolyl, 3- oder 5-Isoxazolyl, 2- oder 3-Pyrrolyl, 2- oder 3-Pyridazinyl, 2-, 4- oder 5-Pyrimidinyl, 2-Pyrazinyl, 2-(1,3,5-Triazinyl), 2- oder 5-Benzimidazolyl, 2-Benzothiazolyl, 1,2,4-Triazol-3-yl, 1,2,4-Triazol-5-yl, Tetrazol-5-yl, Indol-3-yl, Indol-5-yl oder N-Methyl-imidazol-2-, 4- oder -5-yl sein kann und der Arylrest oder Heteroarylrest bis zu zweifach mit F, Cl, Br, OH, CF₃, NO₂, CN, OCF₃, O-(C₁-C₆)-Alkyl, Pyrrolidin-1-yl, Morpholin-1-yl, Piperidin-1-yl, Piperazin-1-yl, 4-Methyl-piperazin-1-yl substituiert sein kann,
sowie deren physiologisch verträgliche Salze.

Weiterhin ganz besonders bevorzugt sind Verbindungen der Formel I, in denen ein oder mehrere Rest(e), die folgende Bedeutung hat bzw. haben:
- Y: eine direkte Bindung;
- X: CH₂;
- R1: Cl;
- R1: H;
- R2: H;
- R3: H;
- R4: Phenyl;
sowie deren physiologisch verträgliche Salze.

Die Erfindung bezieht sich auf Verbindungen der Formel I, in Form ihrer Racemate, racemischen Mischungen und reinen Enantiomere sowie auf ihre Diastereomere und Mischungen davon.

Die Alkyl-, Alkenyl- und Alkinylreste in den Substituenten R1, R1', R2, R3 und R4 können sowohl geradkettig wie verzweigt sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-. Zitronen-, Ethansulfon-, Fumar-, Glucon-, Glykol-, Isethion-, Milch-, Lactobion-, Malein-, Äpfel-, Methansulfon-, Bernstein-, p-Toluolsulfon-, Wein- und Trifluoressigsäure. Für medizinische Zwecke wird in besonders bevorzugter Weise das Chlorsalz verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze, Solvate wie hierin beschrieben.

Die Menge einer Verbindung gemäß Formel (I), die erforderlich ist, um den gewünschten biologischen Effekt zu erreichen. ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht des vom Salz abgeleiteten Dihydrothiazolium-lons. Zur Prophylaxe oder Therapie der oben genannten Zustände können die Verbindungen gemäß Formel (I) selbst als Verbindung verwendet werden, vorzugsweise iiegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformuiierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Ölin-Wasser- oder Wasser-in-Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wäßrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel (I) mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wäßrigen Lösung, gelöst und/oder dispergiert in einem Haftmittel oder dispergiert in einem Polymer. Eine geeignete WirkstoffKonzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Gegenstand der Erfindung ist weiterhin ein Verfahren zur Herstellung der Verbindungen der allgemeinen Formel I, dadurch gekennzeichnet, daß man die Verbindungen der Formel I so gewinnt, daß gemäß dem folgenden Reaktionsschema vorgegangen wird:

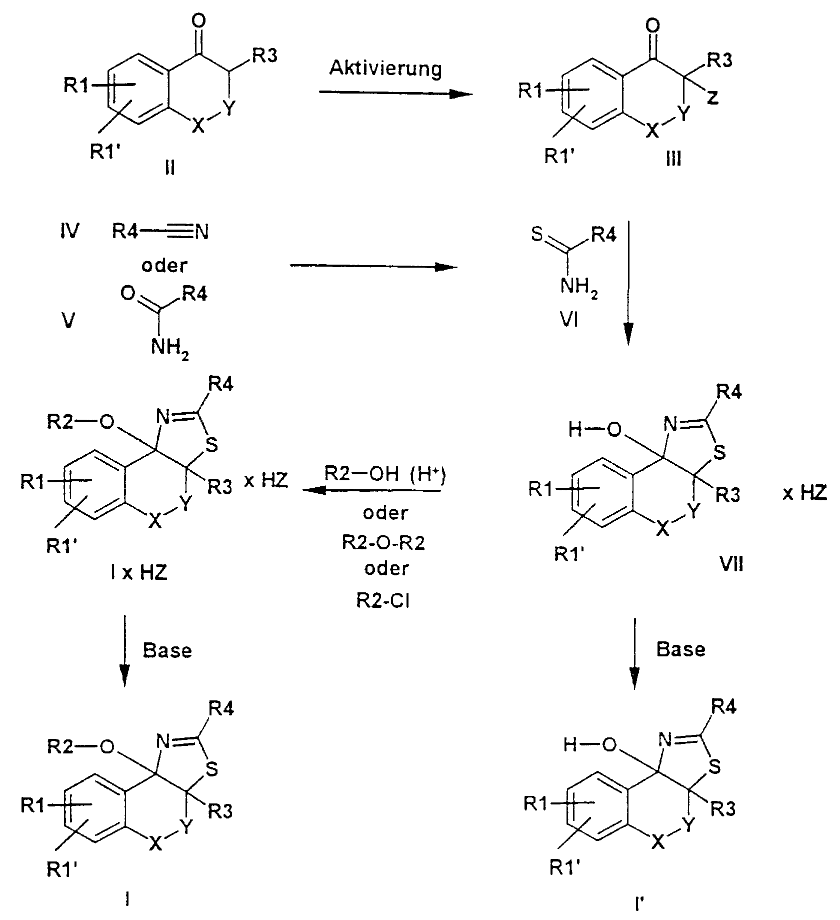

Dazu werden Verbindungen der allgemeinen Formel II, 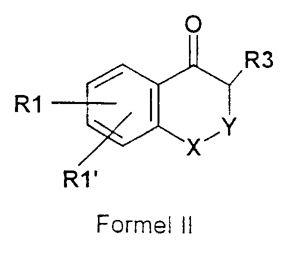 worin R1, R1', R3 und X und Y die angegebene Bedeutung besitzen, aktiviert und in eine Verbindung der Formel III überführt, worin Z für den Rest eines aktivierten Esters einer anorganischen oder organischen Säure steht.
Die Verbindungen der Formel III werden weiter mit Thioamiden der Formel VI 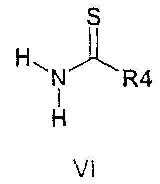 worin R4 die angegebene Bedeutung besitzt, zu Verbindungen der Formel VII bzw. I' umgesetzt, wobei gegebenenfalls die Verbindungen der allgemeinen Formel I' mit organischen oder anorganischen Säuren in ihre Säureadditionssalze der Formel VII oder erhaltene Salze der Formel VII mit organischen oder anorganischen Basen in die freien basischen Verbindungen der Formel I' überführt werden.

Als anorganische Säuren kommen beispielsweise in Betracht:
Halogenwasserstoffsäuren wie Chlorwasserstoffsäure und Bromwasserstoffsäure, sowie Schwefelsäure, Phosphorsäure und Amidosulfonsäure.

Als organische Säuren seien beispielsweise genannt: Ameisensäure, Essigsäure, Benzoesäure, p-Toluolsulfonsäure, Benzolsulfonsäure, Bernsteinsäure, Fumarsäure, Maleinsäure, Milchsäure, Weinsäure, Zitronensäure, L-Ascorbinsäure, Salizylsäure, Isäthionsäure, Methansulfonsäure, Trifluormethansulfonsäure, 1,2-Benzisothiazol-3(2H)-on, 6-Methyl-1,2,3-oxathiazin-4(3H)-on-2,2-dioxid.

Die oben beschriebene Verfahrensweise wird vorteilhaft so ausgeführt, daß man die Verbindungen III mit den Thioamiden VI im molaren Verhältnis von 1:1 bis 1:1,5 umsetzt. Die Reaktion wird vorteilhaft in einem inerten Lösemittel, z.B. in polaren organischen Lösemitteln wie Dimethylformamid, Dimethylacetamid, N-Methyl-2-pyrrolidon, Dioxan, Tetrahydrofuran, Acetonitril, Nitromethan oder Diethylenglykoldimethylether durchgeführt. Als besonders vorteilhafte Lösemittel erweisen sich jedoch Essigsäuremethylester und Essigsäureethylester, kurzkettige Alkohole wie Methanol, Ethanol, Propanol, Isopropanol, sowie niedere Dialkylketone, wie z.B. Aceton, Butan-2-on oder Hexan-2-on. Auch Gemische der angeführten Reaktionsmedien können angewandt werden; so wie auch Gemische der aufgeführten Lösemittel mit Solventien, die für sich alleine genommen weniger geeignet sind, verwendet werden können, wie z. B. Gemische aus Methanol mit Benzol, Ethanol mit Toluol, Methanol mit Diethylether oder mit tert.
Butylmethylether, Ethanol mit Tetrachlormethan, Aceton mit Chloroform, Dichlormethan oder 1,2-Dichlorethan, wobei das jeweils polarere Lösemittel zweckmäßigerweise im Überschuß verwendet werden soll. Die Reaktionspartner können im jeweiligen Reaktionsmedium suspendiert oder gelöst vorliegen. Grundsätzlich können die Reaktionspartner auch ohne Lösemittel umgesetzt werden, insbesondere dann, wenn das jeweilige Thioamid einen möglichst tiefen Schmelzpunkt hat. Die Reaktion verläuft nur wenig exotherm und kann zwischen - 10°C und 150°C, bevorzugt zwischen 30°C und 100°C, durchgeführt werden. Als besonders günstig erwies sich in der Regel ein Temperaturbereich zwischen 50°C und 90°C.

Die Reaktionsdauer ist weitgehend von der Reaktionstemperatur abhängig und liegt zwischen 2 Minuten und 3 Tagen bei höheren bzw. niedrigeren Temperaturen. Im günstigen Temperaturbereich liegt die Reaktionsdauer im allgemeinen zwischen 5 Minuten und 48 Stunden.

Häufig scheiden sich die Verbindungen VII in Form ihrer Säureadditionssalze im Verlauf der Reaktion schwerlöslich ab, zwechmäßig wird nachträglich noch ein geeignetes Fällungsmittel zugesetzt. Als solches verwendet man z.B. Kohlenwasserstoffe wie Benzol, Toluol, Cyclohexan oder Heptan oder Tetrachlormethan; insbesondere erwiesen sich Essigsäurealkylester wie Essigsäureethylester oder Essigsäure-n-butylester oder Dialkylether wie Diethylether, Diisopropylether, Di-n-butylether oder tert.Butylmethylether als besonders geeignet. Bleibt nach Ende der Reaktion das Reaktionsgemisch in Lösung, so kann man die Salze der Verbindungen VII, ggf. nach Konzentrierung der Reaktionslösung, mit einem der genannten Fällungsmittel ausfällen. Ferner kann man die Lösung des Reaktionsgemisches auch vorteilhaft in die Lösung eines der genannten Fällungsmittel unter Rühren einfiltrieren. Da die Reaktion der Verbindungen III mit den Thioamiden VI praktisch quantitativ abläuft, sind die erhaltenen Rohprodukte meistens bereits analytisch rein. Die Aufarbeitung des Reaktionsgemisches kann auch so durchgeführt werden, daß die Reaktionsmischung unter Zusatz einer organischen Base, wie z.B. Triethylamin oder Diisobutylamin oder Ammoniak oder Morpholin oder Piperidin oder 1,8-Diazabicyclo[5.4.0]undec-7-en alkalisch gestellt wird, und das Reaktionsrohprodukt nach Konzentrierung chromatographisch, z.B. über eine Kieselgelsäule, gereinigt wird. Als geeignete Elutionsmedien dafür erweisen sich z.B. Gemische von Essigsäureethylester mit Methanol, Gemische von Dichlormethan mit Methanol, Gemische von Toluol mit Methanol oder Essigsäureethylester oder Gemische von Essigsäurethylester mit Kohlenwasserstoffen wie Heptan. Erfolgt die Reinigung des Rohproduktes auf die zuletzt beschriebene Weise, kann aus der so gewonnenen reinen Base der Formel I' ein Säureadditionsprodukt der Formel VII so gewonnen werden, daß man die Base in einem organischen protischen Lösemittel wie Methanol, Ethanol, Propanol oder Isopropanol oder in einem organischen aprotischen Lösemittel wie Essigsäureethylester, Diethylether, Diisopropylether, tert.Butylmethylether, Dioxan, Tetrahydrofuran, Aceton oder Butan-2-on löst oder suspendiert und diese Mischung anschließend mit einer wenigstens äquimolaren Menge einer anorganischen Säure wie z.B. Chlorwasserstoffsäure, gelöst in einem inerten Lösemittel wie z.B. Diethylether oder Ethanol, oder einer anderen der weiter oben genannten anorganischen oder organischen Säuren versetzt.

Die Verbindungen der Formel I' können aus einem inerten, geeigneten Lösemittel wie z.B. Aceton, Butan-2-on, Acetonitril, Nitromethan umkristallisiert werden. Besonders vorteilhaft ist aber die Umfällung aus einem Lösemittel wie z.B. Dimethylformamid, Dimethylacetamid, Nitromethan, Acetonitril, vorzugsweise Methanol oder Ethanol.

Die Reaktion der Verbindungen der Formel II mit den Thioamiden der Formel VI kann auch so durchgeführt werden, daß man zur Reaktionsmischung eine wenigstens äquimolare Menge einer Base, wie z.B. Triethylamin, zufügt und die so erhaltenen Verbindungen l' anschließend gegebenenfalls in ihre Säureadditionsprodukte VII überführt.

In den Verbindungen der Formel III kommen als Rest eines aktivierten Esters Z beispielsweise in Frage: Cl, Br, J, O-C(O)-(C₆H₄)-4-NO₂, O-SO₂-CH₃, O-SO₂-CF₃, O-SO₂-(C₆H₄)-4-CH₃, O-SO₂-C₆H₄.

Die Säureadditionsprodukte VII und I x HZ können durch Behandlung mit Basen zu den Verbindungen der allgemeinen Formeln I und I' umgesetzt werden. Als Basen kommen beispielsweise Lösungen anorganischer Hydroxide, wie Lithium-, Natrium-, Kalium-, Calcium- oder Bariumhydroxid, Carbonate oder Hydrogencarbonate, wie Natrium- oder Kaliumcarbonat, Natrium- oder Kaliumhydrogencarbonat, Ammoniak und Amine. wie Triethylamin, Diisopropylamin, Dicyclohexylamin, Piperidin, Morpholin, Methyldicyclohexylamin in Frage.

Thioamide der allgemeinen Formel VI sind entweder kommerziell erhältlich oder können z.B. durch Umsetzung des entsprechenden Carbonsäureamids V mit Phosphorpentasulfid in Pyridin (R. N. Hurd, G. Delameter, Chem. Rev. 61, 45 (1961)), oder mit Lawesson's Reagenz in Toluol. Pyridin, Hexamethylphosphorsäurtriamid [Scheibye, Pedersen und Lawesson: Bull. Soc. Chim. Belges 87, 229 (1978)], vorzugsweise in einem Gemisch von Tetrahydrofuran mit 1,3-Dimethyl-3,4,5,6-tetrahydro-2(1H)-pyrimidinon oder 1,3-Dimethyl-2-Imidazolidinon gewonnen werden. Hydroxy-, Amino- oder zusätzliche Carbonylfunktionen werden dabei zweckmäßigerweise mit einer wiederabspaltbaren Schutzfunktion, wie z. B. einem Benzyl-, tert. Butyloxycarbonyl-, Benzyloxycarbonylrest geschützt oder in ein, gegebenfalls cyclisches, Acetal überführt. Methoden dazu sind z. B. beschrieben in Th. W. Greene und P. G. M. Wuts, Protective Groups in Organic Synthesis, Second Edition, 1991, John Wiley & Sons, New York. Thioamide der Formel VI sind auch erhältlich, indem man Nitrile der allgemeinen Formel IV 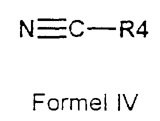 mit Schwefelwasserstoff (Houben-Weyl IX, 762) oder Thioacetamid (E. C. Taylor, J. A. Zoltewicz, J. Am. Chem. Soc. 82, 2656 (1960)) oder O,O-Diethyldithiophosphorsäure umsetzt. Die Umsetzungen mit Schwefelwasserstoff werden vorzugsweise in einem organischen Lösemittel wie Methanol oder Ethanol, die mit Thioacetamid in einem Lösemittel wie Dimethylformamid unter Hinzufügung von Salzsäure, die mit O,O-Diethyl-dithiophosphorsäure in einem Lösemittel wie Essigsäureethylester unter sauren, z.B. HCl, Bedingungen bei Raumtemperatur oder unter Erwärmung durchgeführt.

Die Verbindungen der Formel I x HZ bzw. I, wobei R2 (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl ist, und wobei n = 0 - 5 sein kann, lassen sich gewinnen, indem man entweder
aa) die Säureadditionssalze der Formel VII in einem Lösemittel der Formel R2-OH, wobei R2 die oben beschriebene Bedeutung hat, bei einer Temperatur von -20°C bis 120°C, vorzugsweise bei -5°C bis 50°C, 2 Stunden bis 4 Tage, vorzugsweise 4 Stunden bis 2 Tage, reagieren läßt
   oder
ab) die freien Basen der Formel l'in einem Lösemittel der Formel R2-OH, wobei R2 die oben beschriebene Bedeutung hat, mit äquimolaren, unterschüssigen oder katalytischen, vorzugsweise katalytischen Mengen einer anorganischen oder organischen Säure, wie sie weiter oben beschrieben sind, oder unter Zugabe eines sauren Ionenaustauschers bei einer Temperatur von -20°C bis 120°C, vorzugsweise bei -5°C bis 50°C, 2 Stunden bis 4 Tage, vorzugsweise 4 Stunden bis 2 Tage, umsetzt
   oder
ac) die Umsetzungen nach aa) und ab) in einem inerten aprotischem Lösemittel wie Dichlormethan, Chloroform, 1,2-Dichlorethan, Heptan, Benzol, Toluol, Acetonitril, Nitromethan, Dioxan, Tetrahydrofuran, Ethylenglykoldimethylether, Diethylether, Diisiopropylether, tert.Butylmethylether, Aceton, Butan-2-on oder Essigsäureniederalkylester, wie z.B. Essigsäureethylester, durch Zugabe von 1 bis 5, vorzugsweise 1,5 -2 Äquivalenten einer Verbindung der Formel R2-OH durchführt
   oder
ad) Verbindungen der Formel I' in einem polaren aprotischen Lösemittel, wie z.B. Tetrahydrofuran, Dioxan, Ethylenglykoldimethylether, Nitromethan, Acetonitril oder Dimethylformamid, Dimethylacetamid oder N-Methyl-2-pyrrolidon, mit Hilfe einer Base, wie z.B. Natriumhydrid, Lithiumdiisopropylamid, KOH oder Kaliumcarbonat, in ihr Alkoholat überführt und dieses dann unter Zugabe eines Alkylierungsmittels der Formel R2-W, wobei W = Chlor, Brom, Jod, O-C(O)-CH₃, O-C(O)-CF₃, O-C(O)-C₆H₄-4-NO₂, O-SO₂-CH₃, O-SO₂-CF₃, O-SO₂-C₆H₄-4-CH₃, O-SO₂-C₆H₄-4-NO₂, bei -20 bis 150°C, bevorzugt bei -15 bis 50°C, für 10 Minuten bis 2 Tage, bevorzugt für 20 Minuten bis 12 Stunden, reagieren läßt.
   Verbindungen der Formel I x HZ oder I mit R2 = C(O)-(C₁-C₆)-Alkyl, C(O)-(C₃-C₆)-Cycloalkyl, C(O)-(CH₂)ₙ-Phenyl, C(O)-(CH₂)ₙ-Thienyl, C(O)-(CH₂)ₙ-Pyridyl, C(O)-(CH₂)ₙ-Furyl, wobei n = 0 - 5 sein kann. lassen sich gewinnen, indem man entweder
ba) wie unter aa) - ac) beschrieben vorgeht, mit dem Unterschied, daß man statt eines Alkohols R2-OH eine Säure R2-OH einsetzt, und man 1 bis 2 Äquivalente der Säure R2-OH, vorzugsweise 1,5 Äquivalente der Säure R2-OH einsetzt und auf den Zusatz des unter aa) - ac) beschriebenen sauren anorganischen oder organischen Katalysators verzichtet, den sauren Kationenaustauscher jedoch vorteilhaft einsetzt
   oder
bb) eine Verbindung der Formel VII oder I' mit einer Säure der Formel R2-OH im Sinne einer Mitsunobu Reaktion (O. Mitsunobu, Synthesis 1981, 1) zu einer Verbindung der Formel I x HZ oder I umsetzt
   oder
bc) ein Carbonsäurechlorid der Formel R2-Cl oder ein Carbonsäureanhydrid der Formel R2-O-R2 mit einer Verbindung der Formel I' im Sinne einer Veresterung eines Alkohols (Houben-Weyl, Methoden der Organischen Chemie, Georg Thieme Verlag Stuttgart, Band E5, S. 656-715) umsetzt.

Die nachfolgend aufgeführten Beispiele dienen zur Erläuterung der Erfindung, ohne diese jedoch einzuschränken. Die gemessenen Fest-, bzw. Zersetzungspunkte (Fp.) wurden nicht korrigiert und sind generell von der Aufheizgeschwindigkeit abhängig.

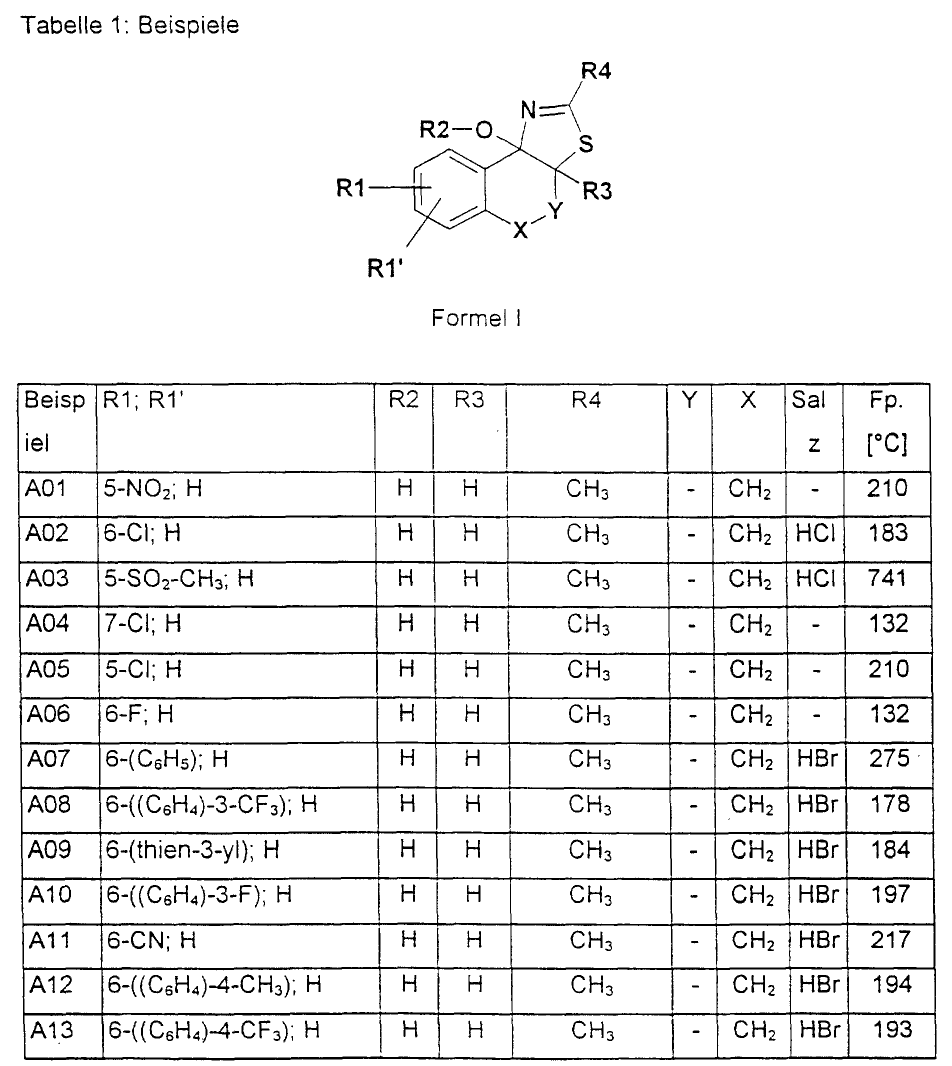

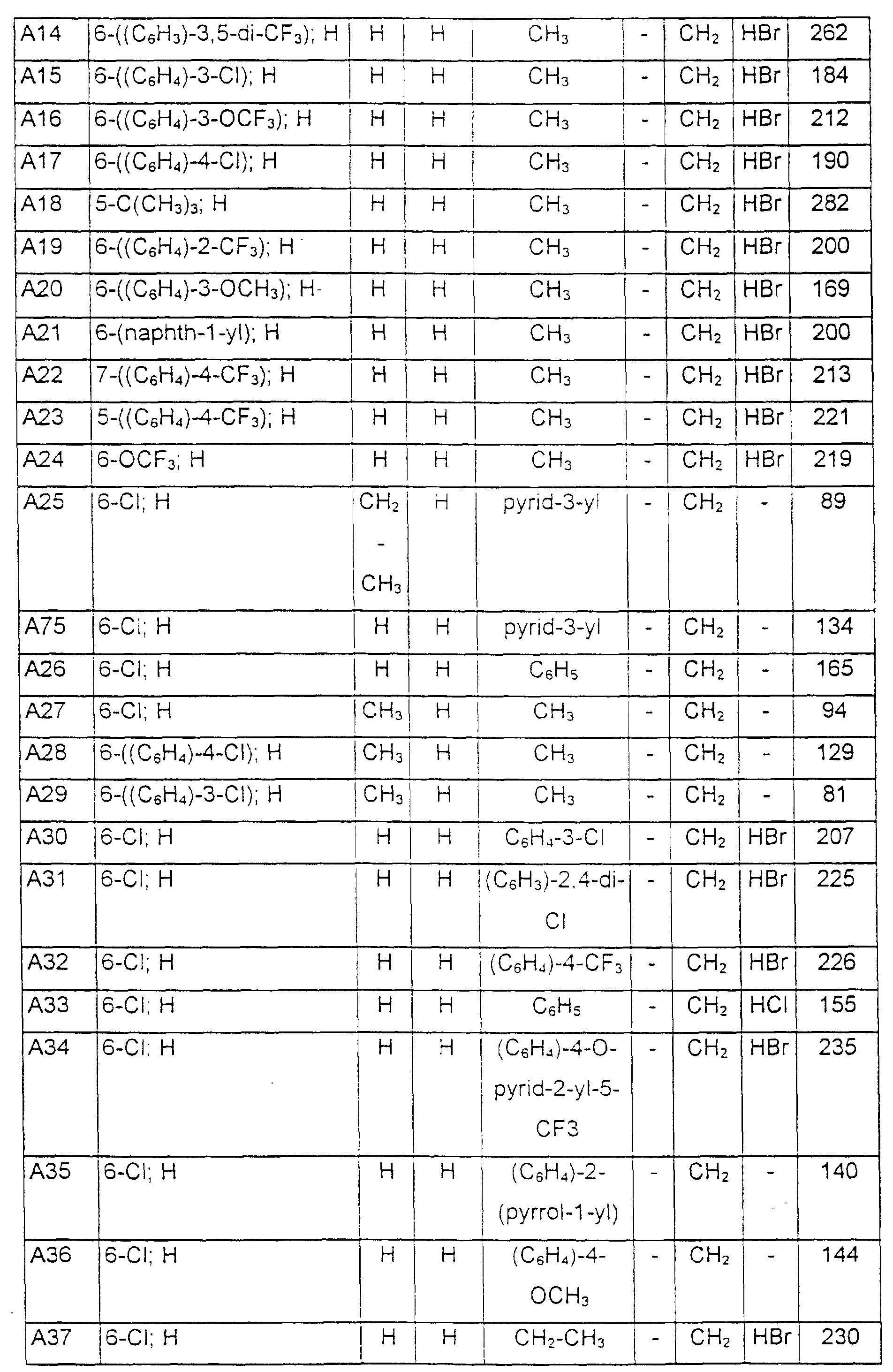

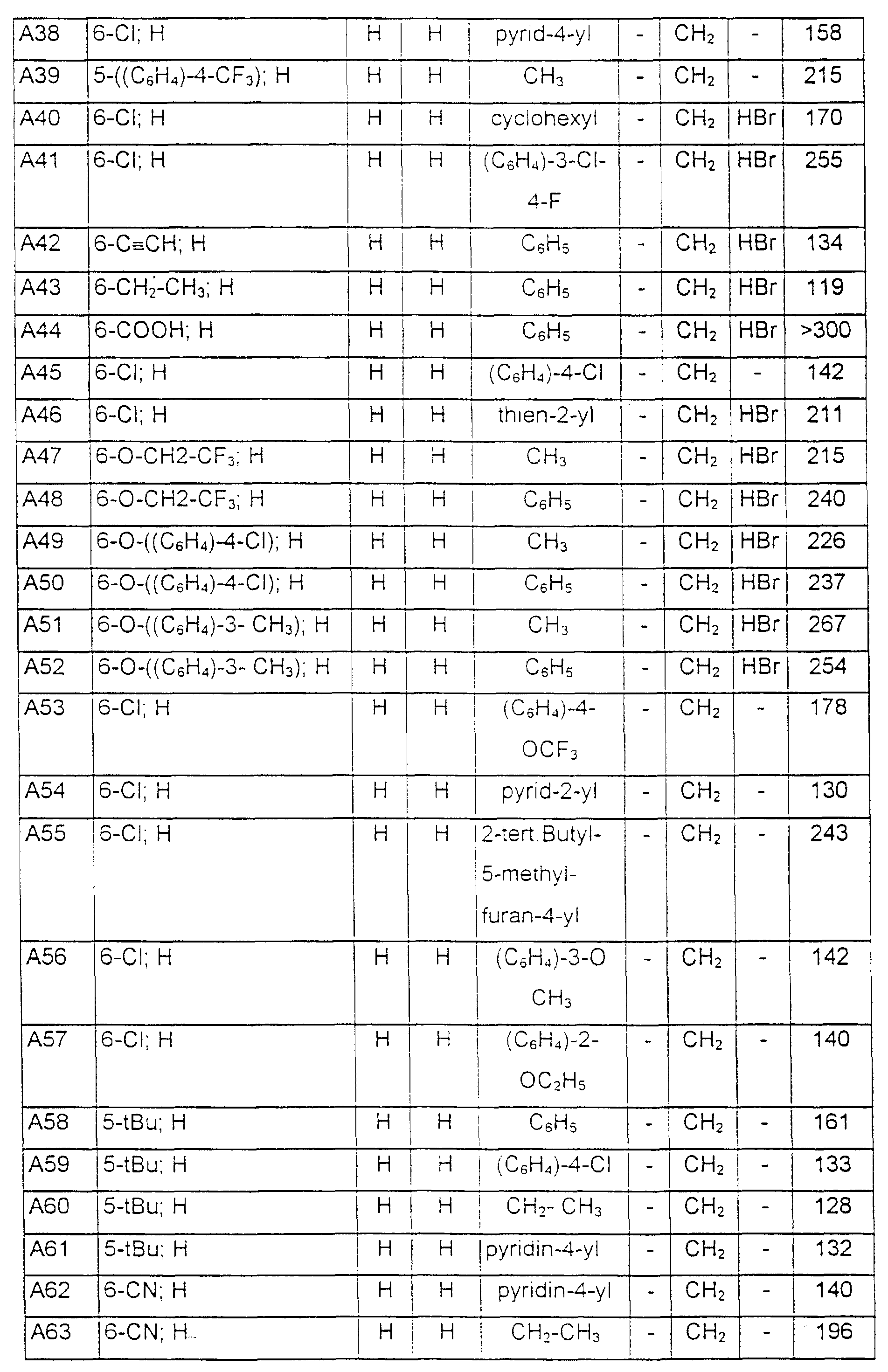

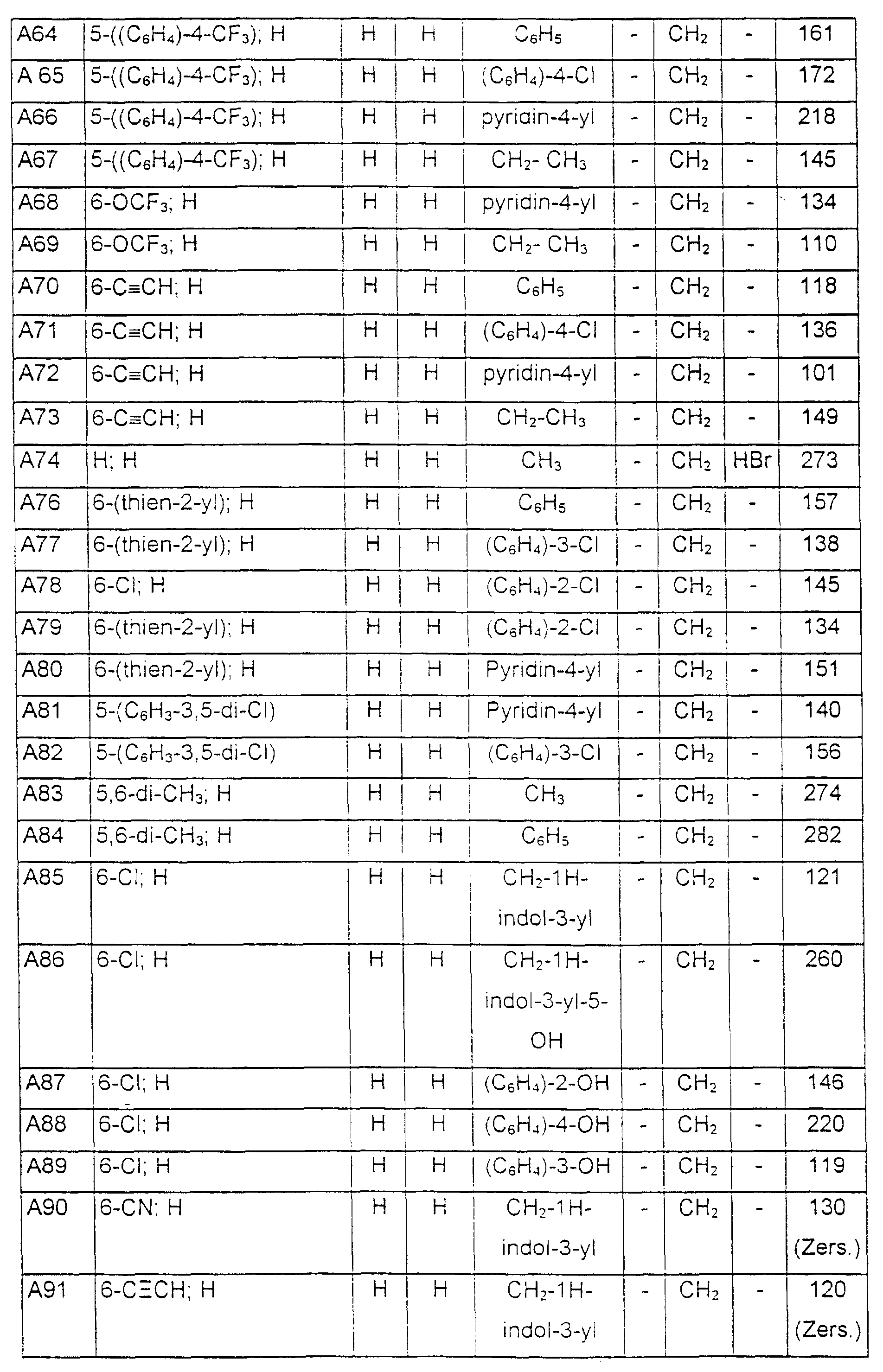

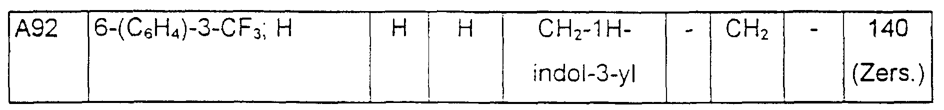

Die Verbindungen der Formel I zeichnen sich durch günstige Wirkungen auf den Fettstoffwechsel aus, insbesondere sind sie als Anorektika/Abmagerungsmittel geeignet. Die Verbindungen können allein oder in Kombination mit weiteren Abmagerungsmitteln eingesetzt werden (wie sie z.B. in Kapitel D1 der Roten Liste beschrieben sind). Die Verbindungen eignen sich zur Prophylaxe sowie insbesondere zur Behandlung von Obesitas. Die Verbindungen eignen sich weiterhin zur Prophylaxe sowie insbesondere zur Behandlung von Typ II Diabetes.

### Die Wirksamkeit der Verbindungen wurde wie folgt getestet:

### Biologisches Prüfmodell:

Die Prüfung der anorektischen Wirkung erfolgte an männlichen NMRI Mäusen. Nach 24stündigem Futterentzug wurde über eine Schlundsonde das Testpräparat verabreicht. In Einzelhaltung und bei freiem Zugang zu Trinkwasser wurde den Tieren 30 Minuten nach Präparatgabe Kondensmilch angeboten. Der Kondensmilchverbrauch wurde halbstündlich 7 Stunden lang bestimmt und das Allgemeinbefinden der Tiere beobachtet. Der gemessene Milchverbrauch wurde mit dem unbehandelter Kontrolltieren verglichen.

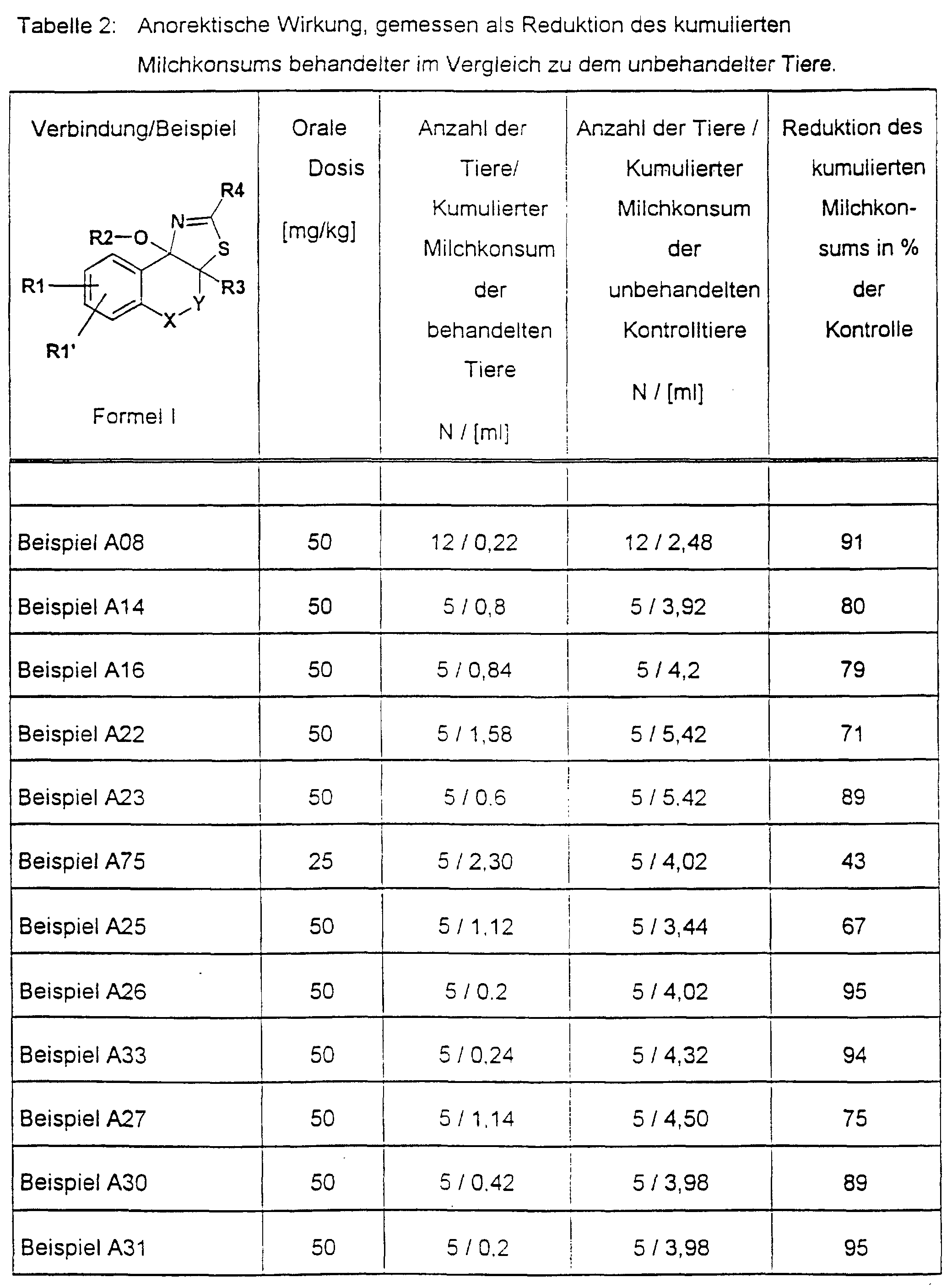

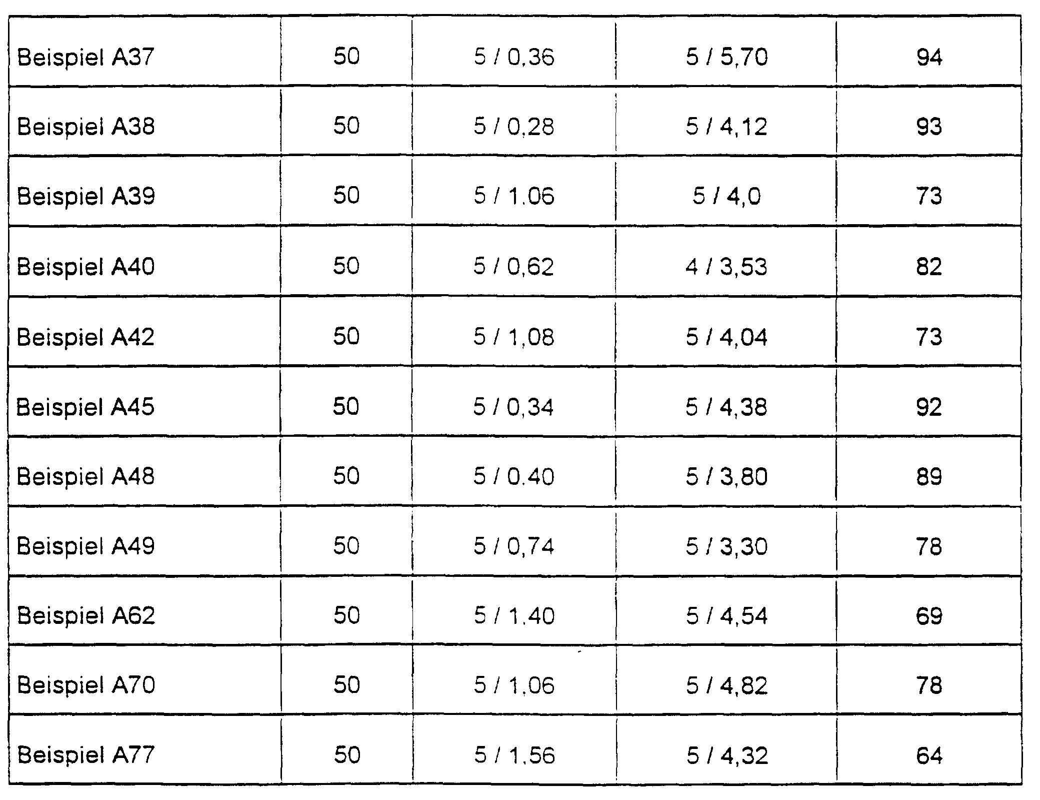

Aus der Tabelle ist abzulesen, daß die Verbindungen der Formel I eine sehr gute anorektische Wirkung zeigen.

Nachfolgend wird die Herstellung einiger Beispiele detailliert beschrieben, die übrigen Verbindungen der Formel I wurden analog erhalten:

### Beispiel 1 (Verbindung A08):

### 2-Methyl-6-(3-trifluormethyl-phenyl)-8,8a-dihydro-indeno[1,2-d]thiazol-3a-ol Hydrobromid:

a) 5-(3-Trifluormethyl-phenyl)-indan-1-on:
   1,5 g 5-Brom-indan-1-on, 1,35 g 3-(Trifluormethyl)-phenylboronsäure und 1,5 g Natriumcarbonat werden in einer Mischung aus 50 ml Toluol mit 10 ml Ethanol und 10 ml Wasser unter Rühren suspendiert. Unter einer Schutzgasatmosphäre (Argon) gibt man 80 mg Palladium-II-acetat und 186 mg Triphenylphosphin zu und rührt die Mischung 3 Stunden unter Rückfluß. Von der abgekühlten Reaktionsmischung wird das Ethanol im Vakuum abdestilliert, und der Rückstand wird mit 20 ml 0,5 N Natronlauge versetzt, gerührt und filtriert. Die organische Phase des Filtrats wird mehrmals mit jeweils 20 ml Wasser und schließlich mit 20 ml ges. Kochsalzlösung gewaschen, über Magnesiumsulfat getrocknet und eingeengt. Der Rückstand wird über Kieselgel mit n-Heptan / Essigsäureethylester 3 / 1 chromatographiert und man erhält 5-(3-Trifluormethylphenyl)-indan-1-on mit dem Schmelzpunkt 93°C.
b) 2-Brom-5-(3-Trifluormethylphenyl)-indan-1-on:
   1,79 g 5-(3-Trifluormethylphenyl)-indan-1-on werden in 10 ml Eisessig gelöst. Man fügt 5,5 µl einer 48%igen Lösung von Bromwasserstoffsäure in Wasser zu und tropft anschließend langsam eine Lösung von 0,362 ml Brom in 5 ml Eisessig zu. Die Reaktionsmischung wird 3 Stunden bei Raumtemperatur gerührt und danach in eine Mischung aus 50 ml Wasser mit 50 g Eis und 86 mg Natriumhydrogencarbonat eingegossen. Die wäßrige Suspension wird mit 100 ml Dichlormethan ausgeschüttelt, die organische Phase dreimal mit 50 ml Wasser gewaschen, über Magnesiumsulfat getrocknet. eingeengt und über Kieselgel mit Toluol / Essigsäureethylester 25 / 1 chromatographiert. Man erhält 2-Brom-5-(3-Trifluoromethylphenyl)-indan-1-on mit dem Schmelzpunkt 96°C. Als Nebenprodukt wird 2,2-Dibrom-5-(3-Trifluoromethylphenyl)-indan-1-on mit dem Schmelzpunkt 210°C (Zers.) isoliert.
c) 2-Methyl-6-(3-trifluoromethyl-phenyl)-8,8a-dihydro-indeno[1,2-d]thiazol-3a-ol Hydrobromid:
   213 mg 2-Brom-5-(3-Trifluoromethylphenyl)-indan-1-on und 48,8 mg Thioacetamid werden in 10 ml trockenem Aceton gelöst und 8 h bei 0°C gerührt. Der Niederschlag wird abgesaugt, mit Aceton gewaschen und im Hochvakuum getrocknet. Man erhält 2-Methyl-6-(3-trifluoromethyl-phenyl)-8,8a-dihydroindeno[1,2-d]thiazol-3a-ol Hydrobromid mit dem Schmelzpunkt 245-250°C (Zers.).

### Beispiel 2 (Verbindung A14):

### 6-(3,5-Bis-trifluormethyl-phenyl)-2-methyl-8,8a-dihydro-indeno[1,2-d]thiazol-3a- ol Hydrobromid:

a) 5-(3,5-Bis-trifluormethyl-phenyl)-indan-1-on:
   Diese Verbindung wird analog dem in Beispiel 1a beschriebenen Verfahren unter Einsatz von 3,5-(Bistrifluormethyl)-phenylboronsäure hergestellt; Schmelzpunkt: 121°C.
b) 5-(3,5-Bis-trifluoromethyl-phenyl)-2-brom-indan-1-on:
   Diese Verbindung wird analog dem in Beispiel 1b beschriebenen Verfahren unter Einsatz der Verbindung 2a hergestellt; Schmelzpunkt: 105°C.
c) 6-(3,5-Bis-trifluoromethyl-phenyl)-2-methyl-8,8a-dihydro-indeno[1,2-d]thiazol-3a- ol Hydrobromid:
   Diese Verbindung wird analog dem in Beispiel 1c beschriebenen Verfahren unter Einsatz der Verbindung 2b hergestellt; Schmelzpunkt: 261-264°C (Zers.).

### Beispiel 3 (Verbindung A16):

### 2-Methyl-6-(3-trifluormethoxy-phenyl)-8,8a-dihydro-indeno[1,2-d]thiazol-3a-ol Hydrobromid:

a) 4,4,5,5-Tetramethyl-2-(3-trifluormethoxy-phenyl)-[1,3,2]dioxaborolan;
   2,41 g 3-(Trifluormethoxy)-brombenzol werden in 25 ml Diethylether gelöst und zu einer Suspension von 0,28 g Magnesium in 25 ml Diethylether so zugetropft, daß die Lösung am Sieden bleibt. Nach beendeter Zugabe wird die Mischung 2 h unter Rückfluß gerührt. Die abgekühlte Lösung wird zu einer Lösung von 1,16 ml Borsäuretrimethylester in 25 ml Diethylether bei einer Temperatur < -50°C zugetropft und anschließend 2 h bei Raumtemperatur gerührt. Zu dieser Lösung werden nacheinander 5 ml 40%ige Schwefelsäure und 1,21 g Pinakol zugegeben. Nach 30 Minuten wird das Lösemittel am Rotavapor® entfernt, der Rückstand mit einer gesättigten Natriumhydrogencarbonatlösung neutralisiert und dreimal mit Methyl-tert.Butylether extrahiert. Die organische Phase wird über Magnesiumsulfat getrocknet und im Vakuum eingeengt. Man erhält 4,4,5,5-Tetramethyl-2-(3-trifluoromethoxy-phenyl)-[1,3,2]dioxaborolan, welches ohne weitere Reinigung weiter umgesetzt wird.
b) 5-(3-Trifluormethoxy-phenyl)-indan-1-on:
   Diese Verbindung wird analog dem in Beispiel 1a beschriebenen Verfahren unter Einsatz von 4,4,5,5-Tetramethyl-2-(3-trifluoromethoxy-phenyl)-[1,3,2]dioxaborolan. hergestellt; gelbliches Öl.
c) 2-Brom-4-trifluormethoxy-indan-1-on:
   Diese Verbindung wird analog dem in Beispiel 1b beschriebenen Verfahren unter Einsatz der Verbindung 3b hergestellt; Schmelzpunkt: 64°C. Als Nebenprodukt wird 2,2-Dibrom-4-trifluormethoxy-indan-1-on (gelbliches Öl) isoliert.
d) 2-Methyl-6-(3-trifluormethoxy-phenyl)-8,8a-dihydro-indeno[1,2-d]thiazol-3a-ol Hydrobromid:
   Diese Verbindung wird analog dem in Beispiel 1c beschriebenen Verfahren unter Einsatz der Verbindung 3c hergestellt: Schmelzpunkt: 210-214°C (Zers.).

### Beispiel 4 (Verbindung A22):

### 2-Methyl-7-(4-trifluormethyl-phenyl)-8,8a-dihydro-indeno[1,2-d]thiazol-3a-ol Hydrobromid:

a) 4-(4-Trifluormethyl-phenyl)-indan-1-on:
   Diese Verbindung wird analog dem in Beispiel 1a beschriebenen Verfahren unter Einsatz von 4-Brom-indan-1-on und 4-(Trifluormethyl)-phenylboronsäure hergestellt; Schmelzpunkt: 75-78°C.
b) 2-Brom-4-(4-trifluoromethyl-phenyl)-indan-1-on:
   Diese Verbindung wird analog dem in Beispiel 1b beschriebenen Verfahren unter Einsatz der Verbindung 4a hergestellt; Schmelzpunkt: 102-105°C. Als Nebenprodukt wird wenig der entsprechenden Dibromverbindung erhalten.
c) 2-Methyl-7-(4-trifluormethyl-phenyl)-8,8a-dihydro-indeno[1,2-d]thiazol-3a-ol Hydrobromid:
   Diese Verbindung wird analog dem in Beispiel 1c beschriebenen Verfahren unter Einsatz der Verbindung 4b hergestellt; Schmelzpunkt: 212-215°C (Zers.).

### Beispiel 5 (Verbindung A23):

### 2-Methyl-5-(4-trifluormethyl-phenyl)-8,8a-dihydro-indeno[1,2-d]thiazol-3a-of Hydrobromid:

a) 6-(4-Trifluormethyl-phenyl)-indan-1-on:
   Diese Verbindung wird analog dem in Beispiel 1a beschriebenen Verfahren unter Einsatz von 6-Brom-indan-1-on und 4-(Trifluormethyl)-phenylboronsäure hergestellt und ohne weitere Reinigung weiter umgesetzt.
b) 2-Brom-6-(4-trifluormethyl-phenyl)-indan-1-on:
   Diese Verbindung wird analog dem in Beispiel 1b beschriebenen Verfahren unter Einsatz der Verbindung 5a hergestellt; Schmelzpunkt; 104°C. Als Nebenprodukt wird wenig der entsprechenden Dibromverbindung erhalten (Fp.: 135°C).
c) 2-Methyl-5-(4-trifluormethyl-phenyl)-8,8a-dihydro-indeno[1,2-d]thiazol-3a-ol Hydrobromid:
   Diese Verbindung wird analog dem in Beispiel 1c beschriebenen Verfahren unter Einsatz der Verbindung 5b hergestellt; Schmelzpunkt: 220-223°C (Zers.).

### Beispiel 6 (Verbindung A25):

### 6-Chlor-3a-ethoxy-2-pyridin-3-yl-8,8a-dihydro-3aH-indeno[1,2-d]thiazol:

a) 2-Brom-5-chlor-indan-1-on:
   Diese Verbindung wird analog dem in Beispiel 1b beschriebenen Verfahren unter Einsatz von 5-Chlor-indan-1-on hergestellt; Schmelzpunkt: 94-96°C. Als Nebenprodukt wird wenig der entsprechenden Dibromverbindung erhalten.
b) 6-Chlor-3a-ethoxy-2-pyridin-3-yl-8,8a-dihydro-3aH-indeno[1,2-d]thiazol:
   1 g 2-Brom-5-chlor-indan-1-on und 622 mg Thionicotinsäureamid werden in 10 ml trockenem Ethanol suspendiert, 2h bei Raumtemperatur und 8 h bei 70°C gerührt. Die Reaktionsmischung wird abgekühlt, der Niederschlag wird abgesaugt, mit Ethanol gewaschen, mit wenig Wasser verrührt, erneut abgesaugt und in 50 ml Essigsäureethylester gelöst. Diese Lösung wird mit Tiethylamin alkalisch gestellt, mit 30 ml Wasser versetzt und die organische Phase wird abgetrennt, mit Wasser gewaschen, über Magnesiumsulfat getrocknet, im Vakuum eingeengt und schließlich an Kieselgel mit Essigsäureethylester / n-Heptan 1 / 1 chromatographiert. Man erhält 6-Chlor-3a-ethoxy-2-pyridin-3-yl-8,8a-dihydro-3aHindeno[1,2-d]thiazol mit dem Schmelzpunkt 89-90°C.

### Beispiel 7 (Verbindung A75):

6-Chlor-2-pyridin-3-yl-8,8a-dihydro-indeno[1,2-d]thiazol-3a-ol mit dem Schmelzpunkt 134-135°C wird neben 6-Chlor-3a-ethoxy-2-pyridin-3-yl-8,8adihydro-3aH-indeno[1,2-d]thiazol bei der Chromatographie des Beispiels 6b erhalten.

### Beispiel 8 (Verbindung A26):

### 6-Chlor-2-phenyl-8,8a-dihydro-indeno[1,2-d]thiazol-3a-ol:

Diese Verbindung wird analog dem in Beispiel 6b beschriebenen Verfahren unter Einsatz von 2-Brom-5-chlor-indan-1-on und Thiobenzamid hergestellt; Schmelzpunkt: 155°C.

### Beispiel 9 (Verbindung A33):6-Chlor-2-phenyl-8,8a-dihydro-indeno[1,2-d]thiazol-3a-ol Hydrochlorid:

a) 6-Chlor-2-phenyl-8,8a-dihydro-indeno[1,2-d]thiazol-3a-ol Hydrobromid:
   Diese Verbindung wird analog dem in Beispiel 1c beschriebenen Verfahren unter Einsatz von 2-Brom-5-chlor-indan-1-on und Thiobenzamid hergestellt; Schmelzpunkt: 245°C.
b) 6-Chlor-2-phenyl-8,8a-dihydro-indeno[1,2-d]thiazol-3a-ol;
   Eine Suspension der Verbindung aus dem Beispiel 9a in Essigsäureethylester wird mit einer gesättigten wäßrigen Natriumhydrogencarbonatlösung versetzt und gerührt. Die organische Phase wird abgetrennt, zweimal mit ges. Natriumhydrogencarbonatlösung gewaschen, über Magnesiumsulfat getrocknet, im Vakuum eingeengt und im Hochvakuum getrocknet. Man erhält 9b mit dem Schmelzpunkt 155°C.
c) 6-Chlor-2-phenyl-8,8a-dihydro-indeno[1,2-d]thiazol-3a-ol Hydrochlorid:
   9 g der Verbindung aus 9b werden in 500 ml Essigsäureethylester suspendiert und unter Rühren bei Eisbadtemperatur portionsweise mit einem Überschuß etherischer HCl-Lösung versetzt. Man rührt 1 h bei Eisbadtemperatur nach, saugt den Niederschlag ab, wäscht ihn mit Diethylether und trocknet ihn im Hochvakuum. Man erhält 9c mit dem Schmelzpunkt 196-199°C.

### Beispiel 10 (Verbindung A27):

### 6-Chlor-3a-methoxy-2-methyl-8,8a-dihydro-3aH-indeno[1,2-d]thiazol;

a) 6-Chlor-2-methyl-8,8a-dihydro-indeno[1.2-d]thiazol-3a-ol Hydrochlorid:
   Diese Verbindung wird analog dem in Beispiel 9a-c beschriebenen Verfahren unter Einsatz von 2-Brom-5-chlor-indan-1-on und Thioacetamid hergestellt; Schmelzpunkt: 183°C.
b) 2 g der Verbindung des Beispiels 10a werden in 50 ml Methanol eingetragen und 2 Tage bei Raumtemperatur gerührt. Die Reaktionsmischung wird eingeengt und zwischen 200 ml Essigsäureethylester und 200 ml ges. Natriumhydrogencarbonatlösung verteilt. Die organische Phase wird abgetrennt, über Natriumsulfat getrocknet, eingeengt und über Kieselgel mit Essigsäureethylester / n-Heptan 1 / 5 chromatographiert. Man erhält 6-Chlor-3a-methoxy-2-methyl-8,8a-dihydro-3aH-indeno[1,2-d]thiazol als wachsartigen Feststoff. ¹H NMR (d₆-dmso, 200 MHz) δ [ppm]= 7,38 (m, 3H), 4,6 (m, 1 H), 3,6 (m, 1 H), 3,22 (s, 3H), 3,02 (m, 1H), 2,2 (s, 3H): MS: MH⁺ = 254.

### Beispiel 11 (Verbindung A30).

### 6-Chlor-2-(3-chlor-phenyl)-8,8a-dihydro-indeno[1,2-d]thiazol-3a-ol Hydrobromid:

Diese Verbindung wird analog dem in Beispiel 1c beschriebenen Verfahren unter Einsatz der von 2-Brom-5-chlor-indan-1-on und 3-Chlor-thiobenzamid hergestellt; Schmelzpunkt: 205-210°C (Zers.).

### Beispiel 12 (Verbindung A31 ).

### 6-Chlor-2-(2,4-dichlor-phenyl)-8,8a-dihydro-indeno[1,2-d]thiazol-3a-ol Hydrobromid:

Diese Verbindung wird analog dem in Beispiel 1c beschriebenen Verfahren unter Einsatz der von 2-Brom-5-chlor-indan-1-on und 2.4-Dichlor-thiobenzamid hergestellt; Schmelzpunkt: 220-230°C (Zers.).

### Beispiel 13 (Verbindung A37).

### 6-Chlor-2-ethyl-8,8a-dihydro-indeno[1,2-d]thiazol-3a-ol Hydrobromid:

Diese Verbindung wird analog dem in Beispiel 1c beschriebenen Verfahren unter Einsatz der von 2-Brom-5-chlor-indan-1-on und Thiopropionamid hergestellt; Schmelzpunkt: 230-240°C (Zers.).

### Beispiel 14 (Verbindung A38).

### 6-Chlor-2-pyridin-4-yl-8,8a-dihydro-indeno[1,2-d]thiazol-3a-ol:

0,98 g 2-Brom-5-chlor-indan-1-on und 0,55 g Thioisonicotinsäureamid werden bei Raumtemperatur in 40 ml trockenem Aceton gelöst und mit 0,55 ml Triethylamin versetzt. Die Mischung rührt man 2 Tage bei Raumtemperatur. Die Reaktionsmischung wird eingeengt, der Rückstand mit Essigsäureethylester aufgenommen und zweimal mit Wasser und danach zweimal mit ges. Kochsalzlösung gewaschen. Die organische Phase wird über Magnesiumsulfat getrocknet, filtriert, eingeengt und über Kieselgel mit Essigsäureethylester / n-Heptan 10 / 1 chromatographiert. Man erhält 6-Chlor-2-pyridin-4-yl-8,8a-dihydroindeno[1,2-d]thiazol-3a-ol mit dem Schmelzpunkt 155-160°C (Zers. ).

### Beispiel 15 (Verbindung A39):

### 2-Methyl-5-(4-trifluormethyl-phenyl)-8,8a-dihydro-indeno[1,2-d]thiazol-3a-ol:

Eine Suspension der Verbindung aus dem Beispiel 5c in Essigsäureethylester wird mit einer gesättigten wäßrigen Natriumhydrogencarbonatlösung versetzt und gerührt. Die organische Phase wird abgetrennt, zweimal mit ges.
Natriumhydrogencarbonatlösung gewaschen, über Magnesiumsulfat getrocknet, im Vakuum eingeengt und im Hochvakuum getrocknet. Man erhält 15 mit dem Schmelzpunkt 213-217°C.

### Beispiel 16 (Verbindung A40):

### 6-Chlor-2-cyclohexyl-8,8a-dihydro-indeno[1,2-d]thiazol-3a-ol Hydrobromid:

Diese Verbindung wird analog dem in Beispiel 1c beschriebenen Verfahren unter Einsatz von 2-Brom-5-chlor-indan-1-on und Cyclohexanthiocarbonsäureamid hergestellt; Schmelzpunkt: 170-175°C (Zers. ).

### Beispiel 17 (Verbindung A40):

### 6-Ethynyl-2-phenyl-8,8a-dihydro-indeno[1,2-d]thiazol-3a-ol Hydrobromid:

a) 5-Ethynyl-indan-1-on:
   2,11 g 5-Brom-indan-1-on werden mit 67,2 mg Palladium-(II)-acetat und 78,6 mg Triphenylphosphin mit 2,12 ml Ethynyl-trimethyl-silan in 10 ml entgastem Triethylamin suspendiert und 1 h unter Rückfluß gerührt. Die abgekühlte Reaktionsmischung wird eingeengt, mit 20 ml ges.
   Natriumhydrogencarbonatlösung und 40 ml Essigsäureethylester aufgenommen und die organische Phase erst mit Wasser, dann mit ges. Kochsalzlösung gewaschen. Die organische Phase wird über Magnesiumsulfat getrocknet, filtriert, im Vakuum eingeengt und über Kieselgel mit n-Heptan / Essigsäureethylester 3 / 1 chromatographiert. Man erhält 5-Trimethylsilanylethynyi-indan-1-on, welches in 25 ml Methanol gelöst und mit Kaliumcarbonat 2 h bei Raumtemperatur gerührt wird. Die Reaktionsmischung wird im Vakuum eingeengt; der Rückstand wird mit 25 ml ges.
   Natriumhydrogencarbonatlösung und 50 ml Essigsäureethylester verrührt, und anschließend wird die organische Phase abgetrennt und über eine Klärschicht filtriert. Das Filtrat wird über Magnesiumsulfat getrocknet, im Vakuum eingeengt und mit n-Heptan verrührt. Der Niederschlag wird abgesaugt und im Vakuum getrocknet. Man erhält 5-Ethynyl-indan-1-on mit dem Schmelzpunkt 157°C.
b) 2-Brom-5-ethynyl-indan-1-on:
   Diese Verbindung wird analog dem in Beispiel 1b beschriebenen Verfahren unter Einsatz der Verbindung 17a hergestellt; Schmelzpunkt: 144°C.
c) 6-Ethynyl-2-phenyl-8,8a-dihydro-indeno[1,2-d]thiazol-3a-ol Hydrobromid:
   Diese Verbindung wird analog dem in Beispiel 1c beschriebenen Verfahren unter Einsatz von 2-Brom-5-ethynyl-indan-1-on und Thiobenzamid hergestellt; Schmelzpunkt: 133-134°C (Zers. ).

### Beispiel 18 (Verbindung A45):

### 6-Chlor-2-(4-chlor-phenyl)-8,8a-dihydro-indeno[1,2-d]thiazol-3a-ol:

Diese Verbindung wird analog dem Vorgehen bei Beispiel 14 unter Einsatz von 2-Brom-5-chlor-indan-1-on und 4-Chlorthiobenzamid gewonnen; Schmelzpunkt: 140-144°C.

### Beispiel 19 (Verbindung A48):

### 2-Phenyl-6-(2,2,2-trifluorethoxy)-8,8a-dihydro-indeno[1,2-d]thiazol-3a-ol Hydrobromid:

a) 5-(2,2,2-Trifluorethoxy)-indan-1-on:
   2,2 ml 2,2,2-Trifluorethanol werden zu einem rührenden Gemisch aus 3,5 g 5-Fluorindanon, 20 ml wasserfreiem Dimethylformamid und 4,1 g wasserfreien und gemahlenen Kaliumkarbonat gegeben und 10 Stunden bei 80°C gerührt. Man destilliert das Lösungsmittel unter verminderten Druck ab , löst den Rückstand in Ethylacetat und wäscht die organische Phase mehrmals mit Wasser. Man erhält das Indanonderivat als bräunlich kristallinen Feststoff nach Chromatographie an Kieselgel mit einem Gemisch aus gleichen Teilen Ethylacetat und Toluol als Elutionsmittel. Schmelzpunkt 93 - 97 °C.
b) 2-Brom-5-(2,2,2-trifluorethoxy)-indan-1-on:
   Diese Verbindung erhält man durch Umsetzung von 0,9 g 5-(2,2,2-Trifluorethoxy)-indan-1-on mit 0,2 ml Brom in 25 ml Essigester. Die Verbindung wird ohne weitere Reinigung weiter verwendet.
c) 2-Phenyl-6-(2,2,2-trifluorethoxy)-8,8a-dihydro-indeno[1,2-d]thiazol-3a-ol Hydrobromid:
   Diese Verbindung wird analog dem in Beispiel 1c beschriebenen Verfahren unter Einsatz von 2-Brom-5-(2,2,2-trifluorethoxy)-indan-1-on und Thiobenzamid hergestellt; Schmelzpunkt: 240-246°C.

### Beispiel 20 (Verbindung A49):

### 6-(4-Chlor-phenoxy)-2-methyl-8,8a-dihydro-indeno[1,2-d]thiazol-3a-ol Hydrobromid:

a) 5-(4-Chlor-phenoxy)-indan-1-on:
   2,82 g 4-Chlorphenol werden nach Auflösung in 60 ml wasserfreiem Dimethylacetamid mit 8,2 g wasserfreien und gemahlenen Kaliumkarbonat ½ Stunde bei Raumtemperatur gerührt. Nach Zugabe von 1,5 g 5-Fluorindanon rührt man 10 Stunden bei 120 - 130°C und destilliert nach Abkühlung das Lösungsmittel unter vermindertem Druck ab. Man versetzt den Rückstand mit Wasser und extrahiert mehrmals mit Ethylacetat. Die organische Phase wird mit 2N NaOH und nachfolgend mit Wasser gewaschen, sodann über 15 Minuten nach Zugabe von Aktivkohle gerührt und das Lösungsmittel nach dem Trocknen über wasserfreiem Magnesiumsulfat unter vermindertem Druck abdestilliert. Der teilweise kristalline dunkle Rückstand wird durch Säulenchromatografie an Kieselgel mit einem Laufmittel, bestehend aus gleichen Teilen Ethylacetat und Toluol, gereinigt. Braune Kristalle, Schmelzpunkt 75 - 80 °C.
   2-Brom-5-(4-chlorphenoxy)-indan-1-on:
      Zu einer Lösung von 1,3 g 5-(4-Chlorphenoxy)-indan-1-on in 30 ml Eisessig tropft man etwa ½ ml einer Lösung aus 0,25 ml Brom in 5 ml Eisessig und erwärmt langsam bis zur Entfärbung des Broms bzw. bis zur beginnenden HBr-Entwicklung. Sodann kühlt man ab und tropft bei Raumtemperatur die restliche Brommenge zu, läßt weitere 2 Stunden rühren und destilliert das Lösungsmittel unter vermindertem Druck ab. Das zurückbleibende dunkle Öl wird ohne weitere Reinigung verwendet.
b) 6-(4-Chlor-phenoxy)-2-methyl-8,8a-dihydro-indeno[1,2-d]thiazol-3a-ol Hydrobromid:
   Diese Verbindung wird analog dem in Beispiel 1c beschriebenen Verfahren unter Einsatz von 2-Brom-5-(4-chlorphenoxy)-indan-1-on und Thioacetamid hergestellt; Schmelzpunkt: 225-228°C.

### Beispiel 21 (Verbindung A62):

### 3a-Hydroxy-2-pyridin-4-yl-8,8a-dihydro-3aH-indeno[1,2-d]thiazole-6-carbonitril:

a) 1-Oxo-indan-5-carbonitril:
   9,5 g 5-Brom-indan-1-on und 4,93 g CuCN werden in 10 ml Dimethylformamid suspendiert und 4 Stunden unter Rückfluß gekocht. Zur abgekühlten, dunkelbraunen, viskosen Suspension wird unter Rühren eine Lösung von 18 g Eisen-III-chlorid in 5 ml konz. Salzsäure mit 30 ml Wasser zugetropft und anschließend für 30 Minuten bei 70°C gerührt. Die Reaktionsmischung wird dreimal mit 50 ml Toluol ausgeschüttelt, und die vereinigten organischen Phasen werden mit 50 ml 2N Salzsäure und 50 ml 2 N Natronlauge ausgeschüttelt und dann mit Wasser neutral gewaschen. Der Toluolextrakt wird über Magnesiumsulfat getrocknet, im Vakuum eingeengt, und der Rückstand wird aus n-Heptan umkristallisiert. Man erhält 1-Oxo-indan-5-carbonitril mit dem Schmelzpunkt 123 - 125°C.
b) 2-Brom-1-oxo-indan-5-carbonitril:
   Die Bromierung des 1-Oxo-indan-5-carbonitrils erfolgt so wie beim Beispiel 1b beschrieben und liefert 2-Brom-1-oxo-indan-5-carbonitril mit dem Schmelzpunkt 115 - 118°C.
c) 3a-Hydroxy-2-pyridin-4-yl-8,8a dihydro-3aH-indeno[1,2-d]thiazole-6-carbonitril:
   Diese Verbindung wird analog dem Vorgehen bei Beispiel 14 unter Einsatz von 2-Brom-1-oxo-indan-5-carbonitril und Thioisonicotinamid gewonnen; Schmelzpunkt: 140°C (Zers.)

### Beispiel 22 (Verbindung A67):

### 2-Ethyl-5-(4-trifluormethyl-phenyl)-8,8a-dihydro-indeno[1,2-d]thiazol-3a-ol;

355 mg (1 mMol) der Verbindung aus Beispiel 5b werden mit 2 mMol Thiopropionsäureamid in 5 ml trockenem Aceton gelöst und 24 h bei Raumtemperatur gerührt. Die Reaktionsmischung wird anschließend 2 h bei Eisbadtemperatur weiter gerührt, der Niederschlag abgesaugt und in 20 ml Essigsäureethylester suspendiert, mit 1,5 mMol Triethylamin versetzt und 30 Minuten bei Raumtemperatur gerührt. Man fügt 10 ml Wasser zu, rührt, trennt die organische Phase ab, trocknet sie über Magnesiumsulfat und engt sie im Vakuum ein. Man erhält 2-Ethyl-5-(4-trifluormethyl-phenyl)-8,8a-dihydro-indeno[1,2-d]thiazol-3a-ol mit dem Schmelzpunkt 145°C

### Beispiel 23 (Verbindung A70):

### 6-Ethynyl-2-phenyl-8,8a-dihydro-indeno[1,2-d]thiazol-3a-ol:

Die Verbindung aus dem Beispiel 17c wird in Essigsäureethylester suspendiert, mit Triethylamin neutralisiert, die Essigsäureethylesterphase mit Wasser gewaschen, anschließend über Magnesiumsulfat getrocknet und im Vakuum eingeengt. Man erhält 6-Ethynyl-2-phenyl-8,8a-dihydro-indeno[1,2-d]thiazol-3a-ol mit dem Schmelzpunkt 118°C.

### Beispiel 24 (Verbindung A77):

### 2-(3-Chlor-phenyl)-6-thiophen-2-yl-8.8a-dihydro-indeno[1,2-d]thiazol-3a-ol: 5-Thiophen-2-yl-indan-1-on:

Diese Verbindung wird analog dem in Beispiel 1a beschriebenen Verfahren unter Einsatz von Thiophen-2-boronsäure hergestellt; Schmelzpunkt: 148°C.
a) 2-Brom-5-thiophen-2-yl-indan-1-on:
   Diese Verbindung wird analog dem in Beispiel 1b beschriebenen Verfahren unter Einsatz der Verbindung 24a hergestellt; Schmelzpunkt: 114°C.
b) 2-(3-Chlor-phenyl)-6-thiophen-2-yl-8,8a-dihydro-indeno[1,2-d]thiazol-3a-ol:
   Diese Verbindung wird erhalten, wenn man die Verbindung aus Beispiel 24b mit 3-Chlorthiobenzamid wie bei Beispiel 22 beschrieben, umsetzt.
   Schmelzpunkt: 137°C.

## Patentansprüche

1. Verbindungen der Formel I, 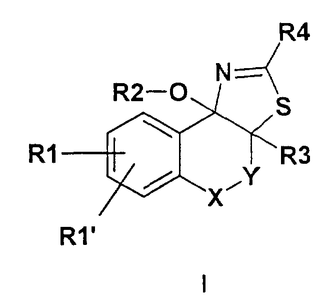 worin bedeuten
Y eine direkte Bindung, -CH₂-, -CH₂-CH₂-:
X CH₂, CH(CH₃), CH(C₂H₅), CH(C₃H₇), CH(C₆H₅);
R1, R1' unabhängig voneinander H, F, Cl, Br, J, CF₃, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können, oder ein Wasserstoff durch OH, OC(O)CH₃, OC(O)H, O-CH₂-Ph, NH₂, NH-CO-CH₃ oder N(COOCH₂Ph)₂ ersetzt sein kann;
SO₂-NH₂, SO₂NH(C₁-C₆)-Alkyl, SO₂N[(C₁-C₆)-Alkyl]₂, S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; NH₂, NH-(C₁-C₆)-Alkyl, N((C₁-C₆)-Alkyl)₂, NH(C₁-C₇)-Acyl, Phenyl, Biphenyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenyl-, Naphthyl-, Pyridyl-, Furanyl- oder Thienylringe jeweils ein bis 3-fach substituiert sein können mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C1-C₆)-Alkyl, CONH₂;
1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
R2 H, (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, C(O)-(C₁-C₆)-Alkyl, C(O)-(C₃-C₆)-Cycloalkyl, C(O)-(CH₂)ₙ-Phenyl, C(O)-(CH₂)ₙ-Thienyl, C(O)-(CH₂)ₙ-Pyridyl, C(O)-(CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C₁-C₆)-Alkyl substituiert sein kann;
R3 H, (C₁-C₆)-Alkyl, F, CN, N₃, O-(C₁-C₆)-Alkyl, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann, (C₂-C₆)-Alkinyl, (C₂-C₆)-Alkenyl, C(O)OCH₃, C(O)OCH₂CH₃, C(O)OH, C(O)NH₂, C(O)NHCH₃, C(O)N(CH₃)₂, OC(O)CH_{3;}
R4 (C₁-C₈)-Alkyl, (C₃-C₇)-Cycloalkyl, (C₂-C₆)-Alkenyl, (C₂-C₆)-Alkinyl, (C₄-C₇)-Cycloalkenyl, wobei in den Alkylresten ein, mehrere, oder alle Wasserstoff(e) durch Fluor ersetzt sein können oder ein Wasserstoff durch OH, OC(O)CH₃, OC(O)H,O-CH₂-Ph oder O-(C₁-C₄)-Alkyl ersetzt sein kann;
(CH₂)n-NR6R7, wobei n = 1-6 sein kann und R6 und R7 unabhängig voneinander H, (C₁-C₆)-Alkyl, (C₃-C₆)-Cycloalkyl, CO-(C₁-C₆)-Alkyl, CHO oder CO-Phenyl sein können, oder -NR6R7 stellt einen Ring wie Pyrrolidin, Piperidin, Morpholin, Piperazin, N-4-Methyl-piperazin-1-yl, N-4-Benzyl-piperazin-1-yl, Phthalimidoyl dar;
(CH₂)ₙ-Aryl, wobei n = 0 - 6 sein kann und Aryl Phenyl, Biphenyl, 1- oder 2-Naphthyl, 2-, 3- oder 4-Pyridyl, 2- oder 3-Thienyl, 2- oder 3-Furyl, 2-, 4- oder 5-Thiazolyl, 2-, 4- oder 5-Oxazolyl, 1-Pyrazolyl, 3- oder 5-Isoxazolyl, 2- oder 3-Pyrrolyl, 2- oder 3-Pyridazinyl, 2-, 4- oder 5-Pyrimidinyl, 2-Pyrazinyl, 2-(1,3,5-Triazinyl), 2- oder 5-Benzimidazolyl, 2-Benzothiazolyl, 1,2,4-Triazol-3-yl, 1,2,4-Triazol-5-yl, Tetrazol-5-yl, Indol-3-yl, Indol-5-yl oder N-Methyl-imidazol-2-, 4- oder -5-yl sein kann und der Arylrest oder Heteroarylrest bis zu zweifach mit F, Cl, Br, OH, CF₃, NO₂, CN, OCF₃, O-(C₁-C₆)-Alkyl, S-(C₁-C₆)-Alkyl, SO-(C₁-C₆)-Alkyl, SO₂-(C₁-C₆)-Alkyl, (C₁-C₆)-Alkyl, (C₃-C₆)-Cycloalkyl, COOH, COO(C₁-C₆)Alkyl, COO(C₃-C₆)Cycloalkyl, CONH₂, CONH(C₁-C₆)Alkyl, CON[(C₁-C₆)Alkyl]2, CONH(C₃-C₆)Cycloalkyl, NH₂, NH-CO-(C₁-C₆)-Alkyl, NH-CO-Phenyl, Pyrrolidin-1-yl, Morpholin-1-yl, Piperidin-1-yl, Piperazin-1-yl, 4-Methyl-piperazin-1-yl, (CH₂)ₙ-Phenyl, O-(CH₂)ₙ-Phenyl, S-(CH₂)ₙ-Phenyl, SO₂-(CH₂)ₙ-Phenyl, wobei n = 0-3, substituiert sein kann;
sowie deren physiologisch verträgliche Salze.

2. Verbindungen der Formel I, gemäß Anspruch 1, **dadurch gekennzeichnet, daß** darin bedeuten
Y eine direkte Bindung;
X CH₂;
R1, R1' unabhängig voneinander H, F, Cl, Br, J, CF₃, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können, oder ein Wasserstoff durch OH, OC(O)CH₃,
OC(O)H, O-CH₂-Ph, NH₂, NH-CO-CH₃ oder N(COOCH₂Ph)₂ ersetzt sein kann;
SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; NH₂, NH-(C₁-C₆)-Alkyl, N((C₁-C₆)-Alkyl)₂, NH(C₁-C₇)-Acyl, Phenyl, Biphenyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenyl-, Naphthyl-, Pyridyl-, Furanyl- oder Thienylringe jeweils ein bis 3-fach substituiert sein können mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C1-C₆)-Alkyl, CONH₂;
1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
R2 H, (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, C(O)-(C₁-C₆)-Alkyl, C(O)-(C₃-C₆)-Cycloalkyl, C(O)-(CH₂)ₙ-Phenyl, C(O)-(CH₂)ₙ-Thienyl, C(O)-(CH₂)ₙ-Pyridyl, C(O)-(CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C₁-C₆)-Alkyl substituiert sein kann;
R3 H, (C₁-C₆)-Alkyl, F, CN, N₃, O-(C₁-C₆)-Alkyl, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C₁-C₆)-Alkyl substituiert sein kann, (C₂-C₆)-Alkinyl, (C₂-C₆)-Alkenyl, C(O)OCH₃, C(O)OCH₂CH₃, C(O)OH, C(O)NH₂, C(O)NHCH₃, C(O)N(CH₃)₂, OC(O)CH₃;
R4 (C₁-C₈)-Alkyl, (C₃-C₇)-Cycloalkyl, (C₂-C₆)-Alkenyl, (C₂-C₆)-Alkinyl, (C₄-C₇)-Cycloalkenyl, wobei in den Alkylresten ein, mehrere, oder alle Wasserstoff(e) durch Fluor ersetzt sein können oder ein Wasserstoff durch OH, OC(O)CH₃, OC(O)H,O-CH₂-Ph oder O-(C₁-C₄)-Alkyl ersetzt sein kann;,
(CH₂)n-NR6R7, wobei n = 1-6 sein kann und R6 und R7 unabhängig voneinander H, (C₁-C₆)-Alkyl, (C₃-C₆)-Cycloalkyl, CO-(C₁-C₆)-Alkyl, CHO oder CO-Phenyl sein können, oder -NR6R7 stellt einen Ring wie Pyrrolidin, Piperidin, Morpholin, Piperazin, N-4-Methyl-piperazin-1-yl, N-4-Benzyl-piperazin-1-yl, Phthalimidoyl dar;
(CH₂)n-Aryl, wobei n = 0 - 6 sein kann und Aryl Phenyl, Biphenyl, 1- oder 2-Naphthyl, 2-, 3- oder 4-Pyridyl, 2- oder 3-Thienyl, 2- oder 3-Furyl, 2-, 4- oder 5-Thiazolyl, 2-, 4- oder 5-Oxazolyl, 1-Pyrazolyl, 3- oder 5-Isoxazolyl, 2- oder 3-Pyrrolyl, 2- oder 3-Pyridazinyl, 2-, 4- oder 5-Pyrimidinyl, 2-Pyrazinyl, 2-(1,3,5-Triazinyl), 2- oder 5-Benzimidazolyl, 2-Benzothiazolyl, 1,2,4-Triazol-3-yl, 1,2,4-Triazol-5-yl, Tetrazol-5-yl, Indol-3-yl, Indol-5-yl oder N-Methyl-imidazol-2-, 4- oder -5-yl sein kann und der Arylrest oder Heteroarylrest bis zu zweifach mit F, Cl, Br, OH, CF₃, NO₂, CN, OCF₃, O-(C₁-C₆)-Alkyl, S-(C₁-C₆)-Alkyl, SO-(C₁-C₆)-Alkyl, SO₂-(C₁-C₆)-Alkyl, (C₁-C₆)-Alkyl, (C₃-C₆)-Cycloalkyl, COOH, COO(C₁-C₆)Alkyl, COO(C₃-C₆)Cycloalkyl, CONH₂, CONH(C₁-C₆)Alkyl, CON[(C₁-C₆)Alkyl]2, CONH(C₃-C₆)Cycloalkyl, NH₂, NH-CO-(C₁-C₆)-Alkyl, NH-CO-Phenyl, Pyrrolidin-1-yl, Morpholin-1-yl, Piperidin-1-yl, Piperazin-1-yl, 4-Methyl-piperazin-1-yl, (CH₂)ₙ-Phenyl, O-(CH₂)ₙ-Phenyl, S-(CH₂)ₙ-Phenyl, SO₂-(CH₂)ₙ-Phenyl, wobei n = 0-3, substituiert sein kann;
sowie deren physiologisch verträgliche Salze.

3. Verbindungen der Formel I gemäß Anspruch 1 oder 2, **dadurch gekennzeichnet, daß** darin bedeuten
Y eine direkte Bindung;
X CH₂;
R1, R1' unabhängig voneinander H, F, Cl, Br, J, CF3, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können, oder ein Wasserstoff durch OH, OC(O)CH₃, OC(O)H, O-CH₂-Ph, NH₂, NH-CO-CH₃ oder N(COOCH₂Ph)₂ ersetzt sein kann;
SO₂-NH₂, SO₂NH(C₁-C₆)-Alkyl, SO₂N[(C₁-C₆)-Alkyl]₂, S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, Biphenyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenyl-, Naphthyl-, Pyridyl-, Furanyl- oder Thienylringe jeweils ein bis 3-fach substituiert sein können mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
R2 H, (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, C(O)-(C₁-C₆)-Alkyl, C(O)-(C₃-C₆)-Cycloalkyl, C(O)-(CH₂)ₙ-Phenyl, C(O)-(CH₂)ₙ-Thienyl, C(O)-(CH₂)ₙ-Pyridyl, C(O)-(CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann;
R3 H, F;
R4 (C₁-C₈)-Alkyl, (C₃-C₇)-Cycloalkyl, (C₂-C₆)-Alkenyl, (C₂-C₆)-Alkinyl, (C₄-C₇)-Cycloalkenyl, wobei in den Alkylresten ein, mehrere, oder alle Wasserstoff(e) durch Fluor ersetzt sein können oder ein Wasserstoff durch OH, OC(O)CH₃, OC(O)H,O-CH₂-Ph oder O-(C₁-C₄)-Alkyl ersetzt sein kann;
(CH₂)n-NR6R7, wobei n = 1-6 sein kann und R6 und R7 unabhängig voneinander H, (C₁-C₆)-Alkyl, (C₃-C₆)-Cycloalkyl, CO-(C₁-C₆)-Alkyl, CHO oder CO-Phenyl sein können, oder -NR6R7 stellt einen Ring wie Pyrrolidin, Piperidin, Morpholin, Piperazin, N4-Methyl-piperazin-1-yl, N4-Benzyl-piperazin-1-yl, Phthalimidoyl dar;
(CH₂)ₙ-Aryl, wobei n = 0 - 6 sein kann und Aryl Phenyl, Biphenyl, 1- oder 2-Naphthyl, 2-, 3- oder 4-Pyridyl, 2- oder 3-Thienyl, 2- oder 3-Furyl, 2-, 4- oder 5-Thiazolyl, 2-, 4- oder 5-Oxazolyl, 1-Pyrazolyl, 3- oder 5-Isoxazolyl, 2- oder 3-Pyrrolyl, 2- oder 3-Pyridazinyl, 2-, 4- oder 5-Pyrimidinyl, 2-Pyrazinyl, 2-(1,3,5-Triazinyl), 2- oder 5-Benzimidazolyl, 2-Benzothiazolyl, 1,2,4-Triazol-3-yl, 1,2,4-Triazol-5-yl, Tetrazol-5-yl, Indol-3-yl, Indol-5-yl oder N-Methyl-imidazol-2-, 4- oder -5-yl sein kann und der Arylrest oder Heteroarylrest bis zu zweifach mit F, Cl, Br, OH, CF₃, NO₂, CN, OCF₃, O-(C₁-C₆)-Alkyl, S-(C₁-C₆)-Alkyl, SO-(C₁-C₆)-Alkyl, SO₂-(C₁-C₆)-Alkyl, (C₁-C₆)-Alkyl, (C₃-C₆)-Cycloalkyl, COOH, COO(C₁-C₆)Alkyl, COO(C₃-C₆)Cycloalkyl, CONH₂, CONH(C₁-C₆)Alkyl, CON[(C₁-C₆)Alkyl]₂, CONH(C₃-C₆)Cycloalkyl, NH₂, NH-CO-(C₁-C₆)-Alkyl, NH-CO-Phenyl, Pyrrolidin-1-yl, Morpholin-1-yl, Piperidin-1-yl, Piperazin-1-yl, 4-Methyl-piperazin-1-yl, (CH2)ₙ-Phenyl, O-(CH₂)ₙ-Phenyl, S-(CH₂)ₙ-Phenyl, SO₂-(CH₂)ₙ-Phenyl, wobei n = 0-3, substituiert sein kann;
sowie deren physiologisch verträgliche Salze.

4. Verbindungen der Formel I, gemäß einen oder mehreren der Ansprüche 1 bis 3, **dadurch gekennzeichnet, daß** darin bedeuten
Y eine direkte Bindung;
X CH₂;
R1, R1' unabhängig voneinander H, F, Cl, Br, NO₂, CN, COOH, (C₁-C₆)-Alkyl, (C₂-C₆)-Alkenyl, (C₂-C₆)-Alkinyl, O-(C₁-C₆)-Alkyl, OCF₃, OCH₂CF₃, Phenyl, O-(CH₂)ₙ-Phenyl, wobei n = 0 - 6 sein kann, 1- oder 2-Naphthyl, 2- oder 3-Thienyl, wobei die Phenyl-, Naphthyl- oder Thienylringe jeweils ein bis 3-fach substituiert sein können mit F, Cl, Br, J, OH, CF₃, NO₂, CN, OCF₃, O-(C₁-C₆)-Alkyl, (C₁-C₆)-Alkyl, NH₂,
R2 H, (C₁-C₆)-Alkyl, (C₃-C₆)-Cycloalkyl, (CH₂)ₙ-Phenyl, wobei n = 0-5 sein kann;
R3 H, F_{;}
R4 (C₁-C₈)-Alkyl, (C₃-C₇)-Cycloalkyl, (CH₂)ₙ-NR6R7, wobei n = 1-6 sein kann und R6 und R7 unabhängig voneinander H, (C₁-C₆)-Alkyl sein können, oder -NR6R7 stellt einen Ring wie Pyrrolidin, Piperidin, Morpholin, Piperazin, N4-Methyl-piperazin-1-yl, N4-Benzyl-piperazin-1-yl, Phthalimidoyl dar;
(CH₂)ₙ-Aryl, wobei n = 0 - 6 sein kann und Aryl Phenyl, Biphenyl, 1- oder 2-Naphthyl, 2-, 3- oder 4-Pyridyl, 2- oder 3-Thienyl, 2- oder 3-Furyl, 2-, 4- oder 5-Thiazolyl, 2-, 4- oder 5-Oxazolyl, 1-Pyrazolyl, 3- oder 5-Isoxazolyl, 2- oder 3-Pyrrolyl, 2- oder 3-Pyridazinyl, 2-, 4- oder 5-Pyrimidinyl, 2-Pyrazinyl, 2-(1,3,5-Triazinyl), 2- oder 5-Benzimidazolyl, 2-Benzothiazolyl, 1,2,4-Triazol-3-yl, 1,2,4-Triazol-5-yl, Tetrazol-5-yl, Indol-3-yl, Indol-5-yl oder N-Methyl-imidazol-2-, 4- oder -5-yl sein kann und der Arylrest oder Heteroarylrest bis zu zweifach mit F, Cl, Br, OH, CF₃, NO₂, CN, OCF₃, O-(C₁-C₆)-Alkyl, Pyrrolidin-1-yl, Morpholin-1-yl, Piperidin-1-yl, Piperazin-1-yl, 4-Methylpiperazin-1-yl;
sowie deren physiologisch verträgliche Salze.

5. Verbindungen der Formel I, gemäß einem oder mehreren der Ansprüche 1 bis 4 **dadurch gekennzeichnet, daß** darin bedeuten
Y eine direkte Bindung;
X CH₂;
R¹ Cl;
R^{1'} H;
R² H;
R³ H;
R⁴ Phenyl;
sowie deren physiologisch verträgliche Salze.

6. Arzneimittel enthaltend eine oder mehrere der Verbindungen gemäß einem oder mehreren der Ansprüche 1 bis 5.

7. Arzneimittel enthaltend eine oder mehrere der Verbindungen gemäß einem oder mehreren der Ansprüche 1 bis 5 und ein oder mehrere Abmagerungsmittel.

8. Verbindungen gemäß einem oder mehreren der Ansprüche 1 bis 5 zur Anwendung als Medikament zur Prophylaxe oder Behandlung der Obesitas.

9. Verbindungen gemäß einem oder mehreren der Ansprüche 1 bis 5 zur Anwendung als Medikament zur Prophylaxe oder Behandlung des Typ II Diabetes.

10. Verbindungen gemäß einem oder mehreren der Ansprüche 1 bis 5 in Kombination mit mindestens einem weiteren anorektischen Wirkstoff zur Anwendung als Medikament zur Prophylaxe oder Behandlung der Obesitas.

11. Verbindungen gemäß einem oder mehreren der Ansprüche 1 bis 5 in Kombination mit mindestens einem weiteren anorektischen Wirkstoff zur Anwendung als Medikament zur Prophylaxe oder Behandlung der des Typ II Diabetes.

12. Verfahren zur Herstellung eines Arzneimittels enthaltend eine oder mehrere der Verbindungen gemäß einem oder mehreren der Ansprüche 1 bis 5, **dadurch gekennzeichnet, daß** der Wirkstoff mit einem pharmazeutisch geeigneten Träger vermischt wird und diese Mischung in eine für die Verabreichung geeignete Form gebracht wird.

13. Verwendung der Verbindungen gemäß einem oder mehreren der Ansprüche 1 bis 5 zur Herstellung eines Medikaments zur Prophylaxe oder Behandlung der Obesitas.

14. Verwendung der Verbindungen gemäß einem oder mehreren der Ansprüche 1 bis 5 zur Herstellung eines Medikaments zur Prophylaxe oder Behandlung des Typ II Diabetes.

## Claims

1. A compound of the formula I 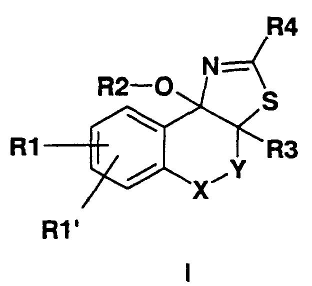 in which
Y is a direct bond, -CH₂-, -CH₂-CH₂-;
X is CH₂, CH(CH₃), CH(C₂H₅), CH(C₃H₇), CH(C₆H₅);
R1, R1' independently of one another are H, F, Cl, Br, I, CF₃, NO₂, CN, COOH, COO(C₁-C₆)alkyl, CONH₂, CONH(C₁-C₆)alkyl, CON[(C₁-C₆)alkyl]₂, (C₁-C₆)-alkyl, (C₂-C₆)-alkenyl, (C₂-C₆)-alkynyl, O-(C₁-C₆)-alkyl, it being possible in the alkyl radicals for one or more, or all hydrogens to be replaced by.fluorine, or a hydrogen to be replaced by OH, OC(O)CH₃, OC(O)H, O-CH₂-Ph, NH₂, NH-CO-CH₃ or N(COOCH₂Ph)₂;
SO₂-NH₂, SO₂NH(C₁-C₆)-alkyl, SO₂N[(C₁-C₆)-alkyl]₂, S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n can be 0 - 6 and the phenyl radical can be substituted up to two times by F, Cl, Br, OH, CF₃, NO₂, CN, OCF₃, O-(C₁-C₆)-alkyl, (C₁-C₆)-alkyl, NH₂; NH₂, NH-(C₁-C₆)-alkyl, N((C₁-C₆)-alkyl)₂, NH(C₁-C₇)-acyl, phenyl, biphenyl, O-(CH₂)ₙ-phenyl, where n can be 0 - 6, 1- or 2-naphthyl, 2-, 3- or 4-pyridyl, 2- or 3-furanyl or 2- or 3-thienyl, where the phenyl, biphenyl, naphthyl, pyridyl, furanyl or thienyl rings can each be substituted one to 3 times by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂; 1,2,3-triazol-5-yl, where the triazole ring can be substituted in the 1-, 2- or 3-position by methyl or benzyl; tetrazol-5-yl, where the tetrazole ring can be substituted in the 1- or 2-position by methyl or benzyl;
R2 is H, (C₁-C₆)-alkyl, (C₃-C₆)-cycloalkyl, (CH₂)ₙ-phenyl, (CH₂)ₙ-thienyl, (CH₂)ₙ-pyridyl, (CH₂)ₙ-furyl, C(O)-(C₁-C₆)-alkyl, C(O)-(C₃-C₆)-cycloalkyl, C(O)-(CH₂)ₙ-phenyl, C(O)-(CH₂)ₙ-thienyl, C(O)-(CH₂)ₙ-pyridyl, C(O)-(CH₂)ₙ-furyl, where n can be 0 - 5 and in which phenyl, thienyl, pyridyl, furyl can each be substituted up to two times by Cl, F, CN, CF₃, (C₁-C₃)-alkyl, OH, O-(C₁-C₆)-alkyl;
R3 is H, (C₁-C₆)-alkyl, F, CN, N₃, O-(C₁-C₆)-alkyl, (CH₂)ₙ-phenyl, (CH₂)ₙ-thienyl, (CH₂)ₙ-pyridyl, (CH₂)ₙ-furyl, where n can be 0 - 5 and in which phenyl, thienyl, pyridyl, furyl can each be substituted up to two times by Cl, F, CN, CF₃, (C₁-C₃)-alkyl, OH, O-(C₁-C₆)-alkyl; (C₂-C₆)-alkynyl, (C₂-C₆)-alkenyl, C(O)OCH₃, C(O)OCH₂CH₃, C(O)OH, C(O)NH₂, C(O)NHCH₃, C(O)N(CH₃)₂, OC(O)CH_{3;}
R4 is (C₁-C₈)-alkyl, (C₃-C₇)-cycloalkyl, (C₂-C₆)-alkenyl, (C₂-C₆)-alkynyl, (C₄-C₇)-cycloalkenyl, it being possible in the alkyl radicals for one or more, or all hydrogens to be replaced by fluorine or a hydrogen to be replaced by OH, OC(O)CH₃, OC(O)H, O-CH₂-Ph or O-(C₁-C₄)-alkyl;
(CH₂)ₙ-NR6R7, where n can be 1-6 and R6 and R7 independently of one another can be H, (C₁-C₆)-alkyl, (C₃-C₆)-cycloalkyl, CO-(C₁-C₆)-alkyl, CHO or CO-phenyl, or -NR6R7 is a ring such as pyrrolidine, piperidine, morpholine, piperazine, N-4-methylpiperazin-1-yl, N-4-benzylpiperazin-1-yl, phthalimidoyl;
(CH₂)ₙ-aryl, where n can be 0-6 and aryl can be phenyl, biphenyl, 1- or 2-naphthyl, 2-, 3- or 4-pyridyl, 2- or 3-thienyl, 2- or 3-furyl, 2-, 4- or 5-thiazolyl, 2-, 4- or 5-oxazolyl, 1-pyrazolyl, 3- or 5-isoxazolyl, 2- or 3-pyrrolyl, 2- or 3-pyridazinyl, 2-, 4- or 5-pyrimidinyl, 2-pyrazinyl, 2-(1,3,5-triazinyl), 2- or 5-benzimidazolyl, 2-benzothiazolyl, 1,2,4-triazol-3-yl, 1,2,4-triazol-5-yl, tetrazol-5-yl, indol-3-yl, indol-5-yl or N-methylimidazol-2-, -4- or -5-yl and the aryl radical or heteroaryl radical can be substituted up to two times by F, Cl, Br, OH, CF₃, NO₂, CN, OCF₃, O-(C₁-C₆)-alkyl, S-(C₁-C₆)-alkyl, SO-(C₁-C₆)-alkyl, SO₂-(C₁-C₆)-alkyl, (C₁-C₆)-alkyl, (C₃-C₆)-cycloalkyl, COOH, COO(C₁-C₆)alkyl, COO(C₃-C₆)cycloalkyl, CONH₂, CONH(C₁-C₆)alkyl, CON[(C₁-C₆)alkyl]2, CONH(C₃-C₆)cycloalkyl, NH₂, NH-CO-(C₁-C₆)-alkyl, NH-CO-phenyl, pyrrolidin-1-yl, morpholin-1-yl, piperidin-1-yl, piperazin-1-yl, 4-methylpiperazin-1-yl, (CH₂)ₙ-pheny), O-(CH₂)ₙ-phenyl, S-(CH₂)ₙ-phenyl, SO₂-(CH₂)ₙ-phenyl, where n = 0-3;
or its physiologically tolerable salts.

2. A compound of the formula I as claimed in claim 1, wherein
Y is a direct bond;
X is CH₂;
R1, R1' independently of one another are H, F, Cl, Br, I, CF₃, NO₂, CN, COOH, COO(C₁-C₆)alkyl, CONH₂, CONH(C₁-C₆)alkyl, CON[(C₁-C₆)alkyl]₂, (C₁-C₆)-alkyl, (C₂-C₆)-alkenyl, (C₂-C₆)-alkynyl, O-(C₁-C₆)-alkyl, it being possible in the alkyl radicals for one or more, or all hydrogens to be replaced by fluorine, or a hydrogen to be replaced by OH, OC(O)CH₃, OC(O)H, O-CH₂-Ph, NH₂, NH-CO-CH₃ or N(COOCH₂Ph)₂;
SO₂-NH₂, SO₂NH(C₁-C₆)-alkyl, SO₂N[(C₁-C₆)-alkyl]₂, S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n can be 0-6 and the phenyl radical can be substituted up to two times by F, Cl, Br, OH, CF₃, NO₂, CN, OCF₃, O-(C₁-C₆)-alkyl, (C₁-C₆)-alkyl, NH₂; NH₂, NH-(C₁-C₆)-alkyl, N((C₁-C₆)-alkyl)₂, NH(C₁-C₇)-acyl, phenyl, biphenyl, O-(CH₂)ₙ-phenyl, where n can be 0-6; 1- or 2-naphthyl, 2-, 3- or 4-pyridyl, 2- or 3-furanyl or 2- or 3-thienyl, where the phenyl, biphenyl, naphthyl, pyridyl, furanyl or thienyl rings can each be substituted one to 3 times by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
1,2,3-triazol-5-yl, where the triazole ring can be substituted in the 1-, 2- or 3-position by methyl or benzyl;
tetrazol-5-yl, where the tetrazole ring can be substituted in the 1- or 2-position by methyl or benzyl;
R2 is H, (C₁-C₆)-alkyl, (C₃-C₆)-cycloalkyl, (CH₂)ₙ-phenyl, (CH₂)ₙ-thienyl, (CH₂)ₙ-pyridyl, (CH₂)ₙ-furyl, C(O)-(C₁-C₆)-alkyl, C(O)-(C₃-C₆)-cycloalkyl, C(O)-(CH₂)ₙ-phenyl, C(O)-(CH₂)ₙ-thienyl, C(O)-(CH₂)ₙ-pyridyl, C(O)-(CH₂)ₙ-furyl, where n can be 0-5 and in which phenyl, thienyl, pyridyl, furyl can each be substituted up to two times by Cl, F, CN, CF₃, (C₁-C₃)-alkyl, OH, O-(C₁-C₆)-alkyl;
R3 is H, (C₁-C₆)-alkyl, F, CN, N₃, O-(C₁-C₆)-alkyl, (CH₂)ₙ-phenyl, (CH₂)ₙ-thienyl, (CH₂)ₙ-pyridyl, (CH₂)ₙ-furyl, where n can be 0-5 and in which phenyl, thienyl, pyridyl, furyl can each be substituted up to two times by Cl, F, CN, CF₃, (C₁-C₃)-alkyl, OH, O-(C₁-C₆)-alkyl; (C₂-C₆)-alkynyl, (C₂-C₆)-alkenyl, C(O)OCH₃, C(O)OCH₂CH₃, C(O)OH, C(O)NH₂, C(O)NHCH₃, C(O)N(CH₃)₂, OC(O)CH_{3;}
R4 is (C₁-C₈)-alkyl, (C₃-C₇)-cycloalkyl, (C₂-C₆)-alkenyl, (C₂-C₆)-alkynyl, (C₄-C₇)-cycloalkenyl, it being possible in the alkyl radicals for one or more, or all hydrogens to be replaced by fluorine or a hydrogen to be replaced by OH, OC(O)CH₃, OC(O)H,O-CH₂-Ph or O-(C₁-C₄)-alkyl;
(CH₂)ₙ-NR6R7, where n can be 1-6 and R6 and R7 independently of one another can be H, (C₁-C₆)-alkyl, (C₃-C₆)-cycloalkyl, CO-(C₁-C₆)-alkyl, CHO or CO-phenyl or -NR6R7 is a ring such as pyrrolidine, piperidine, morpholine, piperazine, N-4-methylpiperazin-1-yl, N-4-benzylpiperazin-1-yl, phthalimidoyl;
(CH₂)ₙ-aryl, where n can be 0-6 and aryl can be phenyl, biphenyl, 1- or 2-naphthyl, 2-, 3- or 4-pyridyl, 2- or 3-thienyl, 2- or 3-furyl, 2-, 4- or 5-thiazolyl, 2-, 4- or 5-oxazolyl, 1-pyrazolyl, 3- or 5-isoxazolyl, 2- or 3-pyrrolyl, 2- or 3-pyridazinyl, 2-, 4- or 5-pyrimidinyl, 2-pyrazinyl, 2-(1,3,5-triazinyl), 2- or 5-benzimidazolyl, 2-benzothiazolyl, 1,2,4-triazol-3-yl, 1,2,4-triazol-5-yl, tetrazol-5-yl, indol-3-yl, indol-5-yl or N-methylimidazol-2-, -4- or -5-yl and the aryl radical or heteroaryl radical can be substituted up to two times by F, Cl, Br, OH, CF₃, NO₂, CN, OCF₃, O-(C₁-C₆)-alkyl, S-(C₁-C₆)-alkyl, SO-(C₁-C₆)-alkyl, SO₂-(C₁-C₆)-alkyl, (C₁-C₆)-alkyl, (C₃-C₆)-cycloalkyl, COOH, COO(C₁-C₆)alkyl, COO(C₃-C₆)cycloalkyl, CONH₂, CONH-(C₁-C₆)alkyl, CON[(C₁-C₆)alkyl]2, CONH(C₃-C₆)cycloalkyl, NH₂, NH-CO-(C₁-C₆)-alkyl, NH-CO-phenyl, pyrrolidin-1-yl, morpholin-1-yl, piperidin-1-yl, piperazin-1-yl, 4-methylpiperazin-1-yl, (CH₂)ₙ-phenyl, O-(CH₂)ₙ-phenyl, S-(CH₂)ₙ-phenyl, SO₂-(CH₂)ₙ-phenyl, where n = 0-3;
or its physiologically tolerable salts.

3. A compound of the formula I as claimed in claim 1 or 2, wherein
Y is a direct bond;
X is CH₂;
R1, R1' independently of one another are H, F, Cl, Br, I, CF3, NO₂, CN, COOH, COO(C₁-C₆)alkyl, CONH₂, CONH(C₁-C₆)alkyl, CON[(C₁-C₆)alkyl]_{2,} (C₁-C₆)-alkyl, (C₂-C₆)-alkenyl, (C₂-C₆)-alkynyl, O-(C₁-C₆)-alkyl, it being possible in the alkyl radicals for one or more, or all hydrogens to be replaced by fluorine, or a hydrogen to be replaced by OH, OC(O)CH₃, OC(O)H, O-CH₂-Ph, NH₂, NH-CO-CH₃ or N(COOCH₂Ph)₂;
SO₂-NH₂, SO₂NH(C₁-C₆)-alkyl, SO₂N[(C₁-C₆)-alkyl]₂, S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n can be 0-6 and the phenyl radical can be substituted up to two times by F, Cl, Br, OH, CF₃, NO₂, CN, OCF₃, O-(C₁-C₆)-alkyl, (C₁-C₆)-alkyl, NH₂;
NH₂, NH-(C₁-C₆)-alkyl, N((C₁-C₆)-alkyl)₂, NH(C₁-C₇)-acyl, phenyl, biphenyl, O-(CH₂)ₙ-phenyl, where n can be 0-6, 1- or 2-naphthyl, 2-, 3- or 4-pyridyl, 2- or 3-furanyl or 2- or 3-thienyl, where the phenyl, biphenyl, naphthyl, pyridyl, furanyl or thienyl rings can each be substituted one to 3 times by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂; 1,2,3-triazol-5-yl, where the triazole ring can be substituted in the 1-, 2- or 3-position by methyl or benzyl;
tetrazol-5-yl, where the tetrazole ring can be substituted in the 1- or 2-position by methyl or benzyl;
R2 is H, (C₁-C₆)-alkyl, (C₃-C₆)-cycloalkyl, (CH₂)ₙ-phenyl, (CH₂)ₙ-thienyl, (CH₂)ₙ-pyridyl, (CH₂)ₙ-furyl, C(O)-(C₁-C₆)-alkyl, C(O)-(C₃-C₆)-cycloalkyl, C(O)-(CH₂)ₙ-phenyl, C(O)-(CH₂)ₙ-thienyl, C(O)-(CH₂)ₙ-pyridyl, C(O)-(CH₂)ₙ-furyl, where n can be 0-5 and in which phenyl, thienyl, pyridyl, furyl can each be substituted up to two times by Cl, F, CN, CF₃, (C₁-C₃)-alkyl, OH, O-(C₁-C₆)-alkyl;
R3 is H, F;
R4 is (C₁-C₈)-alkyl, (C₃-C₇)-cycloalkyl, (C₂-C₆)-alkenyl, (C₂-C₆)-alkynyl, (C₄-C₇)-cycloalkenyl, it being possible in the alkyl radicals for one or more, or all hydrogens to be replaced by fluorine or a hydrogen to be replaced by OH, OC(O)CH₃, OC(O)H, O-CH₂-Ph or O-(C₁-C₄)-alkyl;
(CH₂)ₙ-NR6R7, where n can be 1-6 and R6 and R7 independently of one another can be H, (C₁-C₆)-alkyl, (C₃-C₆)-cycloalkyl, CO-(C₁-C₆)-alkyl, CHO or CO-phenyl, or -NR6R7 is a ring such as pyrrolidine, piperidine, morpholine, piperazine, N4-methylpiperazin-1-yl, N4-benzylpiperazin-1-yl, phthalimidoyl;
(CH₂)ₙ-aryl, where n can be 0-6 and aryl can be phenyl, biphenyl, 1- or 2-naphthyl, 2-, 3- or 4-pyridyl, 2- or 3-thienyl, 2- or 3-furyl, 2-, 4- or 5-thiazolyl, 2-, 4- or 5-oxazolyl, 1-pyrazolyl, 3- or 5-isoxazolyl, 2- or 3-pyrrolyl, 2- or 3-pyridazinyl, 2-, 4- or 5-pyrimidinyl, 2-pyrazinyl, 2-(1,3,5-triazinyl), 2- or 5-benzimidazolyl, 2-benzothiazolyl, 1,2,4-triazol-3-yl, 1,2,4-triazol-5-yl, tetrazol-5-yl, indol-3-yl, indol-5-yl or N-methylimidazol-2-, -4- or -5-yl and the aryl radical or heteroaryl radical can be substituted up to two times by F, Cl, Br, OH, CF₃, NO₂, CN, OCF₃, O-(C₁-C₆)-alkyl, S-(C₁-C₆)-alkyl, SO-(C₁-C₆)-alkyl, SO₂-(C₁-C₆)-alkyl, (C₁-C₆)-alkyl, (C₃-C₆)-cycloalkyl, COOH, COO(C₁-C₆)alkyl, COO(C₃-C₆)-cycloalkyl, CONH₂, CONH(C₁-C₆)alkyl, CON[(C₁-C₆)alkyl]₂, CONH(C₃-C₆)cycloalkyl, NH₂, NH-CO-(C₁-C₆)-alkyl, NH-CO-phenyl, pyrrolidin-1-yl, morpholin-1-yl, piperidin-1-yl, piperazin-1-yl, 4-methylpiperazin-1-yl, (CH₂)ₙ-phenyl, O-(CH₂)ₙ-phenyl, S-(CH₂)ₙ -phenyl, SO₂-(CH₂)ₙ-phenyl, where n = 0-3;
or its physiologically tolerable salts.

4. A compound of the formula I as claimed in one or more of claims 1 to 3, wherein
Y is a direct bond;
X is CH₂;
R1, R1' independently of one another are H, F, Cl, Br, NO₂, CN, COOH, (C₁-C₆)-alkyl, (C₂-C₆)-alkenyl, (C₂-C₆)-alkynyl, O-(C₁-C₆)-alkyl, OCF₃, OCH₂CF₃, phenyl, O-(CH₂)ₙ-phenyl, where n can be 0-6, 1- or 2-naphthyl, 2- or 3-thienyl, where the phenyl, naphthyl or thienyl rings can each be substituted one to 3 times by F, Cl, Br, I, OH, CF₃, NO₂, CN, OCF₃, O-(C₁-C₆)-alkyl, (C₁-C₆)-alkyl, NH₂;
R2 is H, (C₁-C₆)-alkyl, (C₃-C₆)-cycloalkyl, (CH₂)ₙ-phenyl, where n can be 0-5;
R3 is H, F_{;}
R4 is (C₁-C₈)-alkyl, (C₃-C₇)-cycloalkyl, (CH₂)ₙ-NR6R7, where n can be 1-6 and R6 and R7 independently of one another can be H or (C₁-C₆)-alkyl, or -NR6R7 constitutes a ring such as pyrrolidine, piperidine, morpholine, piperazine, N4-methylpiperazin-1-yl, N4-benzylpiperazin-1-yl, phthalimidoyl;
(CH₂)ₙ-aryl, where n can be 0-6 and aryl can be phenyl, biphenyl, 1- or 2-naphthyl, 2-, 3- or 4-pyridyl, 2- or 3-thienyl, 2- or 3-furyl, 2-, 4- or 5-thiazolyl, 2-, 4- or 5-oxazolyl, 1-pyrazolyl, 3- or 5-isoxazolyl, 2- or 3-pyrrolyl, 2- or 3-pyridazinyl, 2-, 4- or 5-pyrimidinyl, 2-pyrazinyl, 2-(1,3,5-triazinyl), 2- or 5-benzimidazolyl, 2-benzothiazolyl, 1,2,4-triazol-3-yl, 1,2,4-triazol-5-yl, tetrazol-5-yl, indol-3-yl, indol-5-yl or N-methylimidazol-2-, -4- or -5-yl and the aryl radical or heteroaryl radical can be substituted up to two times by F, Cl, Br, OH, CF₃, NO₂, CN, OCF₃, O-(C₁-C₆)-alkyl, pyrrolidin-1-yl, morpholin-1-yl, piperidin-1-yl, piperazin-1-yl, 4-methylpiperazin-1-yl;
or its physiologically tolerable salts.

5. A compound of the formula I as claimed in one or more of claims 1 to 4, **characterized in that**
Y is a direct bond;
X is CH₂;
R¹ is Cl;
R^{1'} is H;
R² is H;
R³ is H;
R⁴ is phenyl;
or its physiologically tolerable salts.

6. A pharmaceutical comprising one or more of the compounds as claimed in one or more of claims 1 to 5.

7. A pharmaceutical comprising one or more of the compounds as claimed in one or more of claims 1 to 5 and one or more slimming agents.

8. A compound as claimed in one or more of claims 1 to 5 for use as a medicament for the prophylaxis or treatment of obesity.

9. A compound as claimed in one or more of claims 1 to 5 for use as a medicament for the prophylaxis or treatment of type II diabetes.

10. A compound as claimed in one or more of claims 1 to 5 in combination with at least one further anorectic active compound for use as a medicament for the prophylaxis or treatment of obesity.

11. A compound as claimed in one or more of claims 1 to 5 in combination with at least one further anorectic active compound for use as a medicament for the prophylaxis or treatment of type II diabetes.

12. A process for the production of a pharmaceutical comprising one or more of the compounds as claimed in one or more of claims 1 to 5, which comprises mixing the active compound with a pharmaceutically suitable vehicle and bringing this mixture into a form suitable for administration.

13. The use of the compounds as claimed in one or more of claims 1 to 5 for the production of a medicament for the prophylaxis or treatment of obesity.

14. The use of the compounds as claimed in one or more of claims 1 to 5 for the production of a medicament for the prophylaxis or treatment of type II diabetes.

## Revendications

1. Composés de formule I 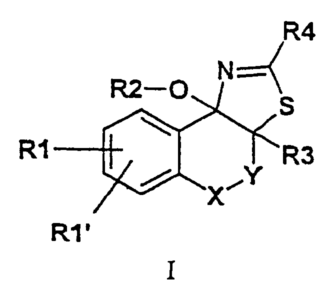 dans laquelle
Y représente une liaison directe ou un groupe -CH₂ ou -CH₂-CH₂-;
X représente un groupe CH₂, CH(CH₃), CH(C₂H₅), CH(C₃H₇), CH(C₆H₅);
R1, R1' représentent, indépendamment l'un de l'autre, H, un reste F, Cl, Br, I, CF₃, NO₂, CN, COOH, COO(alkyle en C₁-C₆), CONH₂, CONH(alkyle en C₁-C₆), CON(alkyle en C₁-C₆)₂, alkyle en C₁-C₆, alcényle en C₂-C₆, alcynyle en C₂-C₆, O-alkyle en C₁-C₆, où, dans les restes alkyle, un, plusieurs ou la totalité des atomes d'hydrogène peuvent être remplacés par du fluor, ou un atome d'hydrogène peut être remplacé par OH, OC(O)CH₃, OC(O)H, O-CH₂-Ph, NH₂, NH-CO-CH₃ ou N(COOCH₂Ph)₂;
un reste SO₂-NH₂, SO₂NH-alkyle en C₁-C₆, SO₂N(alkyle en C₁-C₆)₂, S-alkyle en C₁-C₆, S-(CH₂)ₙ-phényle, SO-alkyle en C₁-C₆, SO-(CH₂)ₙ-phényle, SO₂-alkyle en C₁-C₆, SO₂-(CH₂)ₙ-phényle, n pouvant être égal à 0-6 et le reste phényle pouvant être substitué jusqu'à deux fois par F, Cl, Br, OH, CF₃, NO₂, CN, OCF₃, O-alkyle en C₁-C₆, alkyle en C₁-C₆, NH₂;
un reste NH₂, NH-alkyle en C₁-C₆, N(alkyle en C₁-C₆)₂, NH-acyle en C₁-C₇, phényle, biphényle, O-(CH₂)ₙ-phényle, n pouvant être égal à 0-6, 1-ou 2-naphtyle, 2-, 3- ou 4-pyridyle, 2- ou 3-furanyle ou 2- ou 3-thiényle, les cycles phényle, biphényle, naphtyle, pyridyle, furanyle ou thiényle pouvant chacun être substitués une à trois fois par F, Cl, Br, I, OH, CF₃, NO₂, CN, OCF₃, O-alkyle en C₁-C₆, alkyle en C₁-C₆, NH₂, NH-alkyle en C₁-C₆, N(alkyle en C₁-C₆)₂, SO₂-CH₃, COOH, COO-(alkyle en C₁-C₆), CONH₂;
un reste 1,2,3-triazol-5-yle, le cycle triazole pouvant être substitué en position 1, 2 ou 3 par un reste méthyle ou benzyle;
ou un reste tétrazol-5-yle, le cycle tétrazole pouvant être substitué en position 1 ou 2 par un reste méthyle ou benzyle;
R2 représente H ou un reste alkyle en C₁-C₆, cycloalkyle en C₃-C₆, (CH₂)ₙ-phényle, (CH₂)ₙ-thiényle, (CH₂)ₙ-pyridyle, (CH₂)ₙ-furyle, C(O)-(alkyle en C₁-C₆), C(O)-(cycloalkyle en C₃-C₆), C(O)-(CH₂)ₙ-phényle, C(O)-(CH₂)ₙ-thiényle, C(O)-(CH₂)ₙ-pyridyle, C(O)-(CH₂)ₙ-furyle, où n peut être égal à 0-5 et les restes phényle, thiényle, pyridyle et furyle peuvent chacun être substitués jusqu'à deux fois par Cl, F, CN, CF₃, alkyle en C₁-C₃, OH, O-alkyle en C₁-C₆;
R3 représente H, un reste alkyle en C₁-C₆, F, CN, N₃, O-alkyle en C₁-C₆, (CH₂)ₙ-phényle, (CH₂)ₙ-thiényle, (CH₂)ₙ-pyridyle, (CH₂)ₙ-furyle, où n peut être égal à 0-5 et les restes phényle, thiényle, pyridyle et furyle peuvent chacun être substitués jusqu'à deux fois par Cl, F, CN, CF₃, alkyle en C₁-C₃, OH ou O-alkyle en C₁-C₆; ou un reste alcynyle en C₂-C₆, alcényle en C₂-C₆, C(O)OCH₃, C(O)OCH₂CH₃, C(O)OH, C(O)NH₂, C(O)NHCH₃, C(O)N(CH₃)₂, OC(O)CH₃;
R4 représente un reste alkyle en C₁-C₈, cycloalkyle en C₃-C₇, alcényle en C₂-C₆, alcynyle en C₂-C₆, cycloalcényle en C₄-C₇, où, dans les restes alkyle, un, plusieurs ou la totalité des atomes d'hydrogène peuvent être remplacés par du fluor ou un atome d'hydrogène peut être remplacé par OH, OC(O)CH₃, OC(O)H, O-CH₂-Ph ou O-alkyle en C₁-C₄;
un reste (CH₂)ₙNR6R7 dans lequel n peut être égal à 1-6 et R6 et R7 peuvent être, indépendamment l'un de l'autre, H ou un reste alkyle en C₁-C₆, cycloalkyle en C₃-C₆, CO-alkyle en C₁-C₆, CHO ou CO-phényle, ou NR6R7 représente un cycle comme un cycle pyrrolidine, pipéridine, morpholine, pipérazine, N-4-méthylpipérazin-1-yle, N-4-benzylpipérazin-1-yle, phtalimidoyle;
un reste (CH₂)ₙ-aryle, dans lequel n peut être égal à 0-6 et aryle peut être un reste phényle, biphényle, 1- ou 2-naphtyle, 2-, 3- ou 4-pyridyle, 2- ou 3-thiényle, 2- ou 3-furyle, 2-, 4- ou 5-thiazolyle, 2-, 4- ou 5-oxazolyle, 1-pyrazolyle, 3- ou 5-isoxazolyle, 2- ou 3-pyrrolyle, 2- ou 3-pyridazinyle, 2-, 4- ou 5-pyrimidinyle, 2-pyrazinyle, 2-(1,3,5-triazinyle), 2- ou 5-benzimidazolyle, 2-benzothiazolyle, 1,2,4-triazol-3-yle, 1,2,4-triazol-5-yle, tétrazol-5-yle, indol-3-yle, indol-5-yle ou N-méthylimidazol-2-, -4- ou -5-yle, et le reste aryle ou hétéroaryle peut être substitué jusqu'à deux fois par F, Cl, Br, OH, CF₃, NO₂, CN, OCF₃, O-alkyle en C₁-C₆, S-alkyle en C₁-C₆, SO-alkyle en C₁-C₆, SO₂-alkyle en C₁-C₆, alkyle en C₁-C₆, cycloalkyle en C₃-C₆, COOH, COO(alkyle en C₁-C₆), COO(cycloalkyle en C₃-C₆), CONH₂, CONH(alkyle en C₁-C₆), CON(alkyle en C₁-C₆)₂, CONH(cycloalkyle en C₃-C₆), NH₂, NH-CO-(alkyle en C₁-C₆), NH-CO-phényle, pyrrolidin-1-yle, morpholin-1-yle, pipéridin-1-yle, pipérazin-1-yle, 4-méthylpipérazin-1-yle, (CH₂)ₙ-phényle, O-(CH₂)ₙ-phényle, S-(CH₂)ₙ-phényle, SO₂-(CH₂)ₙ-phényle, n étant égal à 0-3;
et leurs sels physiologiquement acceptables.

2. Composés de formule I selon la revendication 1, **caractérisés en ce que**
Y représente une liaison directe;
X représente un groupe CH₂;
R1, R1' représentent, indépendamment l'un de l'autre, H, un reste F, Cl, Br, I, CF₃, NO₂, CN, COOH, COO(alkyle en C₁-C₆), CONH₂, CONH(alkyle en C₁-C₆), CON(alkyle en C₁-C₆)₂, alkyle en C₁-C₆, alcényle en C₂-C₆, alcynyle en C₂-C₆, O-alkyle en C₁-C₆, où, dans les restes alkyle, un, plusieurs ou la totalité des atomes d'hydrogène peuvent être remplacés par du fluor, ou un atome d'hydrogène peut être remplacé par OH, OC(O)CH₃, OC(O)H, O-CH₂-Ph, NH₂, NH-CO-CH₃ ou N(COOCH₂Ph)₂;
un reste SO₂-NH₂, SO₂NH-alkyle en C₁-C₆, SO₂N(alkyle en C₁-C₆)₂, S-alkyle en C₁-C₆, S-(CH₂)ₙ-phényle, SO-alkyle en C₁-C₆, SO-(CH₂)ₙ-phényle, SO₂-alkyle en C₁-C₆, SO₂-(CH₂)ₙ-phényle, n pouvant être égal à 0-6 et le reste phényle pouvant être substitué jusqu'à deux fois par F, Cl, Br, OH, CF₃, NO₂, CN, OCF₃, O-alkyle en C₁-C₆, alkyle en C₁-C₆, NH₂;
un reste NH₂, NH-alkyle en C₁-C₆, N(alkyle en C₁-C₆)₂, NH-acyle en C₁-C₇, phényle, biphényle, O-(CH₂)ₙ-phényle, n pouvant être égal à 0-6, 1-ou 2-naphtyle, 2-, 3- ou 4-pyridyle, 2- ou 3-furanyle ou 2- ou 3-thiényle, les cycles phényle, biphényle, naphtyle, pyridyle, furanyle ou thiényle pouvant chacun être substitués une à trois fois par F, Cl, Br, I, OH, CF₃, NO₂, CN, OCF₃, O-alkyle en C₁-C₆, alkyle en C₁-C₆, NH₂, NH-alkyle en C₁-C₆, N(alkyle en C₁-C₆)₂, SO₂-CH₃, COOH, COO-(alkyle en C₁-C₆), CONH₂;
un reste 1,2,3-triazol-5-yle, le cycle triazole pouvant être substitué en position 1, 2 ou 3 par un reste méthyle ou benzyle;
ou un reste tétrazol-5-yle, le cycle tétrazole pouvant être substitué en position 1
ou 2 par un reste méthyle ou benzyle;
R2 représente H ou un reste alkyle en C₁-C₆, cycloalkyle en C₃-C₆, (CH₂)ₙ-phényle, (CH₂)ₙ-thiényle, (CH₂)ₙ-pyridyle, (CH₂)ₙ-furyle, C(O)-(alkyle en C₁-C₆), C(O)-(cycloalkyle en C₃-C₆), C(O)-(CH₂)ₙ-phényle, C(O)-(CH₂)ₙ-thiényle, C(O)-(CH₂)ₙ-pyridyle, C(O)-(CH₂)ₙ-furyle, où n peut être. égal à 0-5 et les restes phényle, thiényle, pyridyle et furyle peuvent chacun être substitués jusqu'à deux fois par Cl, F, CN, CF₃, alkyle en C₁-C₃, OH, O-alkyle en C₁-C₆;
R3 représente H, un reste alkyle en C₁-C₆, F, CN, N₃, O-alkyle en C₁-C₆, (CH₂)ₙ-phényle, (CH₂)ₙ-thiényle, (CH₂)ₙ-pyridyle, (CH₂)ₙ-furyle, où n peut être égal à 0-5 et les restes phényle, thiényle, pyridyle et furyle peuvent chacun être substitués jusqu'à deux fois par Cl, F, CN, CF₃, alkyle en C₁-C₃, OH ou O-alkyle en C₁-C₆; ou un reste alcynyle en C₂-C₆, alcényle en C₂-C₆, C(O)OCH₃, C(O)OCH₂CH₃, C(O)OH, C(O)NH₂, C(O)NHCH₃, C(O)N(CH₃)₂, OC(O)CH₃;
R4 représente un reste alkyle en C₁-C₈, cycloalkyle en C₃-C₇, alcényle en C₂-C₆, alcynyle en C₂-C₆, cycloalcényle en C₄-C₇, où, dans les restes alkyle, un, plusieurs ou la totalité des atomes d'hydrogène peuvent être remplacés par du fluor ou un atome d'hydrogène peut être remplacé par OH, OC(O)CH₃, OC(O)H, O-CH₂-Ph ou O-alkyle en C₁-C₄;
un reste (CH₂)ₙNR6R7 dans lequel n peut être égal à 1-6 et R6 et R7 peuvent être, indépendamment l'un de l'autre, H ou un reste alkyle en C₁-C₆, cycloalkyle en C₃-C₆, CO-alkyle en C₁-C₆, CHO ou CO-phényle, ou NR6R7 représente un cycle comme un cycle pyrrolidine, pipéridine, morpholine, pipérazine, N-4-méthylpipérazin-1-yle, N-4-benzylpipérazin-1-yle, phtalimidoyle;
un reste (CH₂)ₙ-aryle, dans lequel n peut être égal à 0-6 et aryle peut être un reste phényle, biphényle, 1- ou 2-naphtyle, 2-, 3- ou 4-pyridyle, 2- ou 3-thiényle, 2- ou 3-furyle, 2-, 4- ou 5-thiazolyle, 2-, 4- ou 5-oxazolyle, 1-pyrazolyle, 3- ou 5-isoxazolyle, 2- ou 3-pyrrolyle, 2- ou 3-pyridazinyle, 2-, 4- ou 5-pyrimidinyle, 2-pyrazinyle, 2-(1,3,5-triazinyle), 2- ou 5-benzimidazolyle, 2-benzothiazolyle, 1,2,4-triazol-3-yle, 1,2,4-triazol-5-yle, tétrazol-5-yle, indol-3-yle, indol-5-yle ou N-méthylimidazol-2-, -4- ou -5-yle, et le reste aryle ou hétéroaryle peut être substitué jusqu'à deux fois par F, Cl, Br, OH, CF₃, NO₂, CN, OCF₃, O-alkyle en C₁-C₆, S-alkyle en C₁-C₆, SO-alkyle en C₁-C₆, SO₂-alkyle en C₁-C₆, alkyle en C₁-C₆, cycloalkyle en C₃-C₆, COOH, COO(alkyle en C₁-C₆), COO(cycloalkyle en C₃-C₆), CONH₂, CONH(alkyle en C₁-C₆), CON(alkyle en C₁-C₆)₂, CONH(cycloalkyle en C₃-C₆), NH₂, NH-CO-(alkyle en C₁-C₆), NH-CO-phényle, pyrrolidin-1-yle, morpholin-1-yle, pipéridin-1-yle, pipérazin-1-yle, 4-méthylpipérazin-1-yle, (CH₂)ₙ-phényle, O-(CH₂)ₙ-phényle, S-(CH₂)ₙ-phényle, SO₂-(CH₂)ₙ-phényle, n étant égal à 0-3;
et leurs sels physiologiquement acceptables.

3. Composés de formule I selon la revendication 1 ou 2, **caractérisés en ce que**
Y représente une liaison directe;
X représente un groupe CH₂;
R1, R1' représentent, indépendamment l'un de l'autre, H, un reste F, Cl, Br, I, CF₃, NO₂, CN, COOH, COO(alkyle en C₁-C₆), CONH₂, CONH(alkyle en C₁-C₆), CON(alkyle en C₁-C₆)₂, alkyle en C₁-C₆, alcényle en C₂-C₆, alcynyle en C₂-C₆, O-alkyle en C₁-C₆, où, dans les restes alkyle, un, plusieurs ou la totalité des atomes d'hydrogène peuvent être remplacés par du fluor, ou un atome d'hydrogène peut être remplacé par OH, OC(O)CH₃, OC(O)H, O-CH₂-Ph, NH₂, NH-CO-CH₃ ou N(COOCH₂Ph)₂;
un reste SO₂-NH₂, SO₂NH-alkyle en C₁-C₆, SO₂N(alkyle en C₁-C₆)₂, S-alkyle en C₁-C₆, S-(CH₂)ₙ-phényle, SO-alkyle en C₁-C₆, SO-(CH₂)ₙ-phényle, SO₂-alkyle en C₁-C₆, SO₂-(CH₂)ₙ-phényle, n pouvant être égal à 0-6 et le reste phényle pouvant être substitué jusqu'à deux fois par F, Cl, Br, OH, CF₃, NO₂, CN, OCF₃, O-alkyle en C₁-C₆, alkyle en C₁-C₆, NH₂;
un reste NH₂, NH-alkyle en C₁-C₆, N(alkyle en C₁-C₆)₂, NH-acyle en C₁-C₇, phényle, biphényle, O-(CH₂)ₙ-phényle, n pouvant être égal à 0-6, 1-ou 2- naphtyle, 2-, 3- ou 4-pyridyle, 2- ou 3-furanyle ou 2- ou 3-thiényle, les cycles phényle, biphényle, naphtyle, pyridyle, furanyle ou thiényle pouvant chacun être substitués une à trois fois par F, Cl, Br, I, OH, CF₃, NO₂, CN, OCF₃, O-alkyle en C₁-C₆, alkyle en C₁-C₆, NH₂, NH-alkyle en C₁-C₆, N(alkyle en C₁-C₆)₂, SO₂-CH₃, COOH, COO-(alkyle en C₁-C₆), CONH₂;
un reste 1,2,3-triazol-5-yle, le cycle triazole pouvant être substitué en position 1, 2 ou 3 par un reste méthyle ou benzyle;
ou un reste tétrazol-5-yle, le cycle tétrazole pouvant être substitué en position 1 ou 2 par un reste méthyle ou benzyle;
R2 représente H ou un reste alkyle en C₁-C₆, cycloalkyle en C₃-C₆, (CH₂)ₙ-phényle, (CH₂)ₙ-thiényle, (CH₂)ₙ-pyridyle, (CH₂)ₙ-furyle, C(O)-(alkyle en C₁-C₆), C(O)-(cycloalkyle en C₃-C₆), C(O)-(CH₂)ₙ-phényle, C(O)-(CH₂)ₙ-thiényle, C(O)-(CH₂)ₙ-pyridyle, C(O)-(CH₂)ₙ-furyle, où n peut être égal à 0-5 et les restes phényle, thiényle, pyridyle et furyle peuvent chacun être substitués jusqu'à deux fois par Cl, F, CN, CF₃, alkyle en C₁-C₃, OH, O-alkyle en C₁-C₆;
R3 représente H ou F;
R4 représente un reste alkyle en C₁-C₈, cycloalkyle en C₃-C₇, alcényle en C₂-C₆, alcynyle en C₂-C₆, cycloalcényle en C₄-C₇, où, dans les restes alkyle, un, plusieurs ou la totalité des atomes d'hydrogène peuvent être remplacés par du fluor ou un atome d'hydrogène peut être remplacé par OH, OC(O)CH₃, OC(O)H, O-CH₂-Ph ou O-alkyle en C₁-C₄;
un reste (CH₂)ₙNR6R7 dans lequel n peut être égal à 1-6 et R6 et R7 peuvent être, indépendamment l'un de l'autre, H ou un reste alkyle en C₁-C₆, cycloalkyle en C₃-C₆, CO-alkyle en C₁-C₆, CHO ou CO-phényle, ou NR6R7 représente un cycle comme un cycle pyrrolidine, pipéridine, morpholine, pipérazine, N-4-méthylpipérazin-1-yle, N-4-benzylpipérazin-1-yle, phtalimidoyle;
un reste (CH₂)ₙ-aryle, dans lequel n peut être égal à 0-6 et aryle peut être un reste phényle, biphényle, 1- ou 2-naphtyle, 2-, 3- ou 4-pyridyle, 2- ou 3-thiényle, 2- ou 3-furyle, 2-, 4- ou 5-thiazolyle, 2-, 4- ou 5-oxazolyle, 1-pyrazolyle, 3- ou 5-isoxazolyle, 2- ou 3-pyrrolyle, 2- ou 3-pyridazinyle, 2-, 4- ou 5-pyrimidinyle, 2-pyrazinyle, 2-(1,3,5-triazinyle), 2- ou 5-benzimidazolyle, 2-benzothiazolyle, 1,2,4-triazol-3-yle, 1,2,4-triazol-5-yle, tétrazol-5-yle, indol-3-yle, indol-5-yle ou N-méthylimidazol-2-, -4- ou -5-yle, et le reste aryle ou hétéroaryle peut être substitué jusqu'à deux fois par F, Cl, Br, OH, CF₃, NO₂, CN, OCF₃, O-alkyle en C₁-C₆, S-alkyle en C₁-C₆, SO-alkyle en C₁-C₆, SO₂-alkyle en C₁-C₆, alkyle en C₁-C₆, cycloalkyle en C₃-C₆, COOH, COO(alkyle en C₁-C₆), COO(cycloalkyle en C₃-C₆), CONH₂, CONH(alkyle en C₁-C₆), CON(alkyle en C₁-C₆)₂, CONH(cycloalkyle en C₃-C₆), NH₂, NH-CO-(alkyle en C₁-C₆), NH-CO-phényle, pyrrolidin-1-yle, morpholin-1-yle, pipéridin-1-yle, pipérazin-1-yle, 4-méthylpipérazin-1-yle, (CH₂)ₙ-phényle, O-(CH₂)ₙ-phényle, S-(CH₂)ₙ-phényle, SO₂-(CH₂)ₙ-phényle, n étant égal à 0-3;
et leurs sels physiologiquement acceptables.

4. Composés de formule I selon l'une ou plusieurs des revendications 1 à 3, **caractérisés en ce que**
Y représente une liaison directe;
X représente un groupe CH₂;
R1, R1' représentent, indépendamment l'un de l'autre, H, un reste F, Cl, Br, NO₂, CN, COOH, alkyle en C₁-C₆, alcényle en C₂-C₆, alcynyle en C₂-C₆, O-alkyle en C₁-C₆, OCF₃, OCH₂CF₃, phényle, O-(CH₂)ₙ-phényle, n pouvant être égal à 0-6, 1- ou 2-naphtyle ou 2- ou 3-thiényle, les cycles phényle, naphtyle ou thiényle pouvant chacun être substitués une à trois fois par F, Cl, Br, I, OH, CF₃, NO₂, CN, OCF₃, O-alkyle en C₁-C₆, alkyle en C₁-C₆, NH₂;
R2 représente H ou un reste alkyle en C₁-C₆, cycloalkyle en C₃-C₆, (CH₂)ₙ-phényle, où n peut être égal à 0-5;
R3 représente H ou F;
R4 représente un reste alkyle en C₁-C₈, cycloalkyle en C₃-C₇, (CH₂)ₙNR6R7 dans lequel n peut être égal à 1-6 et R6 et R7 peuvent être, indépendamment l'un de l'autre, H ou un reste alkyle en C₁-C₆, ou NR6R7 représente un cycle comme un cycle pyrrolidine, pipéridine, morpholine, pipérazine, N-4-méthylpipérazin-1-yle, N-4-benzylpipérazin-1-yle, phtalimidoyle;
un reste (CH₂)ₙ-aryle, dans lequel n peut être égal à 0-6 et aryle peut être un reste phényle, biphényle, 1- ou 2-naphtyle, 2-, 3- ou 4-pyridyle, 2- ou 3- thiényle, 2- ou 3-furyle, 2-, 4- ou 5-thiazolyle, 2-, 4- ou 5-oxazolyle, 1- pyrazolyle, 3- ou 5-isoxazolyle, 2- ou 3-pyrrolyle, 2- ou 3-pyridazinyle, 2-, 4- ou 5-pyrimidinyle, 2-pyrazinyle, 2-(1,3,5-triazinyle), 2- ou 5-benzimidazolyle, 2-benzothiazolyle, 1,2,4-triazol-3-yle, 1,2,4-triazol-5-yle, tétrazol-5-yle, indol-3-yle, indol-5-yle ou N-méthylimidazol-2-, -4- ou -5-yle, et le reste aryle ou hétéroaryle peut être substitué jusqu'à deux fois par F, Cl, Br, OH, CF₃, NO₂, CN, OCF₃, O-alkyle en C₁-C₆, pyrrolidin-1-yle, morpholin-1-yle, pipéridin-1-yle, pipérazin-1-yle, 4-méthylpipérazin-1-yle;
et leurs sels physiologiquement acceptables.

5. Composés de formule I selon l'une ou plusieurs des revendications 1 à 4, **caractérisés en ce que**
Y représente une liaison directe;
X représente un groupe CH₂;
R1 représente Cl;
R1' représente H;
R2 représente H;
R3 représente H;
R4 représente un groupe phényle;
et leurs sels physiologiquement acceptables.

6. Médicament contenant un ou plusieurs des composés selon l'une ou plusieurs des revendications 1 à 5.

7. Médicament contenant un ou plusieurs des composés selon l'une ou plusieurs des revendications 1 à 5 et un ou plusieurs agents amaigrissants.

8. Composés selon l'une ou plusieurs des revendications 1 à 5 à utiliser comme médicaments pour la prophylaxie ou le traitement de l'obésité.

9. Composés selon l'une ou plusieurs des revendications 1 à 5 à utiliser comme médicaments pour la prophylaxie du diabète de type II.

10. Composés selon l'une ou plusieurs des revendications 1 à 5 en combinaison avec au moins une autre substance active anorexigène, à utiliser comme médicaments pour la prophylaxie ou le traitement de l'obésité.

11. Composés selon l'une ou plusieurs des revendications 1 à 5 en combinaison avec au moins une autre substance active anorexigène, à utiliser comme médicaments pour la prophylaxie ou le traitement du diabète de type II.

12. Procédé de préparation d'un médicament contenant un ou plusieurs composés selon l'une ou plusieurs des revendications 1 à 5, **caractérisé en ce que** l'on mélange la substance active avec un support pharmaceutiquement approprié et on met ce mélange sous une forme appropriée à l'administration.

13. Utilisation des composés selon l'une ou plusieurs des revendications 1 à 5 pour la préparation d'un médicament destiné à la prophylaxie ou au traitement de l'obésité.

14. Utilisation des composés selon l'une ou plusieurs des revendications 1 à 5 pour la préparation d'un médicament destiné à la prophylaxie ou au traitement du diabète de type II.
